(19) Europäisches Patentamt
European Patent Office
Office européen des brevets

(11) EP 4 393 493 A1

(12) EUROPEAN PATENT APPLICATION
published in accordance with Art. 153(4) EPC

(43) Date of publication:
**03.07.2024 Bulletin 2024/27**

(21) Application number: **22860658.8**

(22) Date of filing: **26.08.2022**

(51) International Patent Classification (IPC):
***A61K 31/675*** *(2006.01)* ***A61P 35/00*** *(2006.01)*

(52) Cooperative Patent Classification (CPC):
**A61K 31/675; A61P 35/00**

(86) International application number:
**PCT/CN2022/115284**

(87) International publication number:
**WO 2023/025312 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2021 CN 202110996009**

(71) Applicant: **Ascentawits Pharmaceuticals, Ltd.**
**Shenzhen, Guangdong 518118 (CN)**

(72) Inventors:
- **QI, Tianyang**
  **Shenzhen, Guangdong 518118 (CN)**
- **LIU, Xing**
  **Shenzhen, Guangdong 518118 (CN)**
- **DUAN, Jianxin**
  **Shenzhen, Guangdong 518118 (CN)**
- **MENG, Fanying**
  **San Francisco California 94121 (US)**
- **LI, Anrong**
  **Shenzhen, Guangdong 518118 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(54) **PARP INHIBITOR-RESISTANT PATIENT TREATED WITH TH-302**

(57) The present invention provides treatment methods using TH-302 as a monotherapy or in combination therapy to treat a PARP inhibitor-resistant patient, a drug and a pharmaceutical use thereof.

**Capan-1 Cell Line (BRCA-Mutated)**

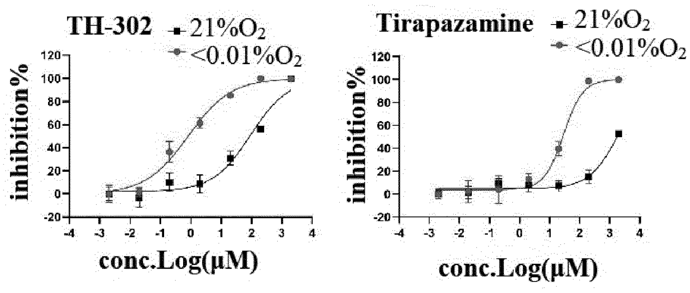


Fig. 1



Processed by Luminess, 75001 PARIS (FR)

## Description

### TECHNICAL FIELD

The present invention relates to methods of treating cancer and, in particular, to a method of treating a PARP inhibitor (PARPi)-resistant cancer patient.

### BACKGROUND

The first human clinical trial of the PARPi drug, olaparib, has demonstrated for the first time that the PARPi can inhibit the growth of BRCA1/2 mutation-carrying tumor cells. This is mainly based on the synthetic lethality theory (Ashworth, A., & Lord, C. J. (2018). Synthetic Lethal Therapies for Cancer: What's Next after PARP Inhibitors? Nature Reviews. Clinical Oncology, 15 (9), 564-576. https://doi.org/10.1038/s41571-018-0055-6): the PARP inhibitor can inhibit PARP's function to repair the single-strand break in DNA, resulting in that lots of single-strand breaks in DNA within a cell fail to be timely repaired. Unrepaired single-strand breaks in DNA trigger collapses of replication forks, and hence cause double-strand breaks in DNA. In healthy cells, double-strand breaks in DNA with high cytotoxicity can be repaired through the homologous recombination repair (HR) pathway which is mediated jointly by BRCA1, BRCA2 and other proteins. However, in a BRCA1/2 deficient tumor cell, double-strand breaks in DNA cannot be repaired, eventually causing death of the tumor cell. The PARPi was initially developed for use in radiation therapy and chemotherapy sensitization, and there were pre-clinical studies in support of developing the PARPi as a monotherapy drug for treating BRCA1/2 gene deficient cancer. For these reasons, germline BRCA1/2 (gBRCA1/2) mutation carriers were selected as the initial target population for verifying the PARPi-BRCA hypothesis. Subjects admitted into the initial study of the PARPi for ovarian cancer all had received platinum-based chemotherapy, and the study found that platinum sensitivity directly correlated with a response to the PARPi (platinum-based chemotherapy drugs are DNA breaking agents and induce DNA crosslinking, which can be partially repaired via the HR pathway; therefore, DNA repair deficient tumors are expected to be sensitive to platinum-based chemotherapy). Two other PARPis have been approved in ovarian cancer: niraparib and rucaparib. Niraparib is FDA- and EMA- approved as maintenance treatment (regardless of BRCA1/2 status), and rucaparib is also registered by the FDA and EMA as optional treatment for BRCA1/2-mutation associated ovarian cancer patients who have received 2 previous lines of chemotherapy. In addition, talazoparib has also been FDA-approved for treatment of BRCA-mutated or HER2-negative metastatic breast cancer (Mateo, J., Lord, C. J., Serra, V., Tutt, A., Balmaña, J., Castroviejo-Bermejo, M., Cruz, C., Oaknin, A., Kaye, S. B., & de Bono, J. S. (2019). A Decade of Clinical Development of PARP Inhibitors in Perspective. Annals of Oncology: official journal of the European Society for Medical Oncology, 30(9), 1437-1447.https://doi.org/10.1093/annonc/mdz192).

As the PARPi is clinically used, PARPi resistance is about to become an inevitable problem in their clinical uses. Over 40% of BRCAm (BRCA-mutated) ovarian cancer patients have failed to benefit from PARPis. Studies to date have demonstrated that homologous recombination repair restoration (HRR), DNA replication fork protection and pharmacokinetic changes of the PARPi are the main causes for PARPi resistance. In order to overcome PARPi resistance and enhance sensitivity of PARPi drugs, various combination therapy regimens are being developed, and many of them have advanced to the clinical stages. These mainly include: PARPi-DNA alkylator combination; PARPi-oncolytic herpes simplex virus (oHSV) combination; PARPi-ion radiotherapy combination; PARPi- immunotherapy combination; PARPi-HSP90 inhibitor combination; PARPi-WEE1/ATR inhibitor combination; PARPi-DNMTi inhibitor combination; PARPi-CDK inhibitor combination; and so forth (He Li, Zhao-Yi Liu, Nayiyuan Wu, Yong-Chang Chen, Quan Cheng and Jing Wang. PARP Inhibitor Resistance: the Underlying Mechanisms and Clinical Implications. Mol Cancer, 2020 Jun 20;19(1): 107.2020. https://doi.org/10.1186/s12943-020-01227-0; Rose, M., Burgess, J. T., O'Byrne, K., Richard, D. J., & Bolderson, E. (2020). PARP Inhibitors: Clinical Relevance, Mechanisms of Action and Tumor Resistance. Frontiers in Cell and Developmental Biology, 8, 564601. https://doi.org/10.3389/fcell.2020.564601).

TH-302 (evofosfamide, CAS No. 918633-87-1) is a 2-nitroimidazole triggered hypoxia activated prodrug (HAP) of bromo-isophosphoramide mustard, developed by the US company Threshold. Under hypoxic conditions, the inactive TH-302 prodrug can release highly toxic Br-IPM. TH-302 possesses a broad spectrum of biological activity in vitro and in vivo, specific hypoxia-selective activation activity, and the activity of inducing H2AX phosphorylation and DNA crossing-linking, leading to cell cycle arrest. Therefore, this compound has been evaluated by many pharmaceutical companies and scientific research institutions for its use in the development of anti-cancer drugs.

As pointed out in a research paper by Meng F Y (Meng Fanying) et al., TH-302 shows broad-spectrum activity against various tumors and provides an excellent hypoxia-selective activity-enhancing effect. A study has shown that TH-302 exhibits significantly higher in vitro cytotoxicity to 32 human cancer cell lines under hypoxia than under normoxia, demonstrating that this compound has selective cytotoxicity to cancer cells in hypoxic environments. Using human cells with overexpressed one-electron reductase (POR), the mechanism of one-electron reductase-dependent activity enhancement by TH-302 under hypoxia has been confirmed, as shown in the following Chemical Reaction Equation 1:

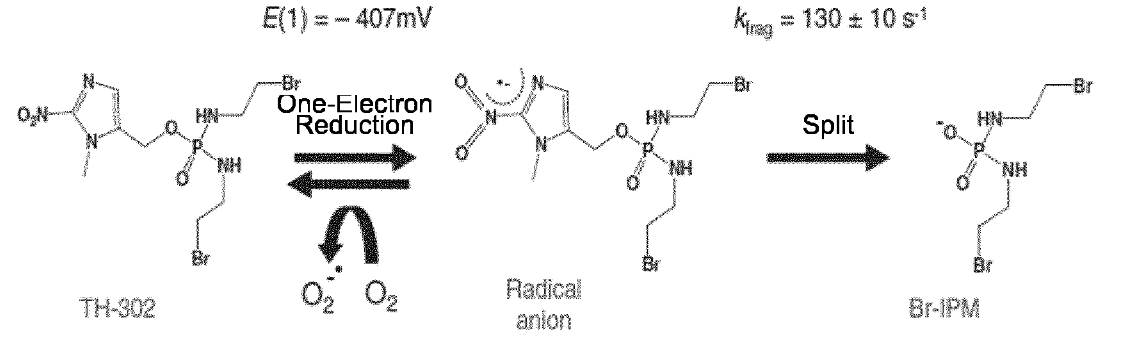


Chemical Reaction Equation 1

Cytochrome P450 oxidoreductase reduces the prodrug TH-302 into an intermediate radical anion, which is unstable. Then, the radical anion functions by being decomposed into Br-IPM, a cytotoxin with cytotoxic effects. The key step of this procedure is the one-electron reduction process. Studies have confirmed that the presence of oxygen will reverse the one-electron reduction process. In other words, the presence of oxygen will hinder the progress of the one-electron reduction process. Therefore, only in a hypoxic environment can TH-302 be reduced to provide stronger cytotoxicity. Further, in vitro cytotoxicity of TH-302 has been assayed using DNA repair mutant cell lines based on Chinese hamster ovary cells, including cell lines deficient in base excision, nucleotide excision or non-homologous end-joining repair, or cell lines deficient in homologous end-joining repair (which are cell lines deficient in homology-dependent repair). Studies find that cell lines deficient in homologous end-joining repair alone, or both homologous end-joining repair and nucleotide excision repair, exhibit marked sensitivity to TH-302 under hypoxia, but the sensitivity of cell lines deficient in base excision, nucleotide excision or non-homologous end-joining repair alone to TH-302 is not affected. Consistent with this finding, enhanced sensitivity to TH-302 has also been observed in in vitro experiments on cells deficient in BRCA1, BRCA2, and FANCA, and better therapeutic effects of TH-302 on patients with BRCA genetic mutations have been observed in clinical trials (Meng F, Evans J W, Bhupathi D, et al. Molecular and Cellular Pharmacology of the Hypoxia-Activated Prodrug TH-302. [J]. Molecular Cancer Therapeutics, 2012, 11(3): 740; Conroy, M., Borad, M. J., & Bryce, A. H. (2017). Hypoxia-Activated Alkylating Agents in BRCA1-Mutant Ovarian Serous Carcinoma. Cureus, 9(7), e1517. https://doi.org/10.7759/cureus.1517; WO2015013448A1, Treatment of Pancreatic Cancer with a Combination of a Hypoxia-Activated Prodrug and a Taxane; WO2020007106A1, Anti-Cancer Medical Use of Evofosfamide).

These studies about the mechanism of action of TH-302, in particular those revealing the fact that TH-302 has special sensitivity to BRCA mutations, suggest the possibility of the drug TH-302 to be used in combination therapy to overcome the resistance problem of a PARPi.

However, in the application PCT/US2012/031677 (Pub. No. WO2012135757A2, entitled "Methods for Treating Cancer", filed by the US company Threshold), researchers of Threshold conducted an in vitro study using TH-302 and the candidate PARPi drug ABT-888 (i.e., veliparib, CAS No. 912444-00-9) as a combination therapy.

Different cancer cells were pretreated with ABT-888 for 1 h under normoxia, and then co-incubated with TH-302 for additional 2 h under either normoxia or hypoxia. After 3 days of incubation in the presence of ABT-888, cell viability was determined using AlamarBlue. The results are shown in the table below.

Results of H460 Cell Line (human large cell lung cancer cells):

| Compound | $IC_{50}$ (μM) | |
|---|---|---|
| | Normoxia | Hypoxia |
| TH-302 | 47 | 0.2 |
| TH-302 + 1 μM ABT888 | 39 | 0.1 |
| TH-302 + 10 μM ABT888 | 36 | 0.2 |

Results of HCT116 Cell Line (human colon cancer cells):

| Compound | $IC_{50}$ (μM) | |
|---|---|---|
| | Normoxia | Hypoxia |
| TH-302 | 61 | 0.1 |
| TH-302 + 0.5 μM ABT888 | 84 | 0.1 |
| TH-302 + 5 μM ABT888 | 85 | 0.2 |

Results of A375 Cell Line (human malignant melanoma cells):

| Compound | $IC_{50}$ (μM) | |
|---|---|---|
| | Normoxia | Hypoxia |
| TH-302 | 230 | 1.5 |
| TH-302 + 0.5 μM ABT888 | 230 | 2.0 |
| TH-302 + 5 μM ABT888 | 210 | 2.0 |

The results demonstrated that the combination therapy of TH-302 with ABT-888 did not have an additive effect in the in vitro cell experiments. That is, TH-302 activity was not substantially affected by the presence of ABT-888.

However, in the application PCT/US2019/065065 (Pub. No. WO2020118251A2, entitled "Hypoxia Targeting Compositions and Combinations Thereof with a PARP Inhibitor and Methods of Use thereof'), it is pointed out that combination therapy of a hypoxia-activated drug or a prodrug thereof (e.g., apaziquone, AQ4N, etanidazole, evofosfamide (TH-302), nimorazole, pimonidazole, porfiromycin, PR-104, tarloxotinib or tirapazamine) with a PARPi has an additional effect. In particular, it also discloses the results of an in vivo animal test of combination therapy of tirapazamine with olaparib, which demonstrate that a combination comprising the hypoxia-activated anti-cancer prodrug tirapazamine with the PARPi olaparib can significantly delay tumor growth in PDX animal models, as compared to monotherapy with tirapazamine or the PARPi. That is, combination therapy of the hypoxia-activated anti-cancer drug with the PARPi has an additive effect.

In other words, there is still a controversy about whether combination therapy of a hypoxia-activated anti-cancer prodrug and a PARPi can provide an additive effect, indicating the complexity of such additive effects.

## SUMMARY

Researchers of the applicant found in an efficacy test that combination therapy of TH-302 with a PARPi exhibited an additive effect in some in vivo tumor growth inhibition assays in animals, which was totally different from the results of in vitro cell experiments conducted by Threshold in 2012. Thus, the applicant conducted additional researches and made an additional surprising finding that TH-302 had an excellent therapeutic effect on PARPi-resistant cancer models even as a monotherapy!

Based on the experimental results, the present application provides the following cancer treatment methods.

A treatment method uses a drug containing a hypoxia activated compound of formula (I) as a monotherapy or in combination therapy to treat a PARPi-resistant cancer or tumor patient:

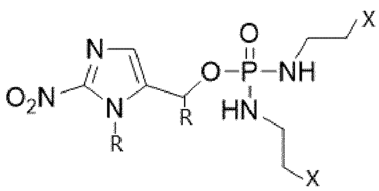

(I),

where each R is independently selected from H, $-CH_3$ and $-CH_2CH_3$, and each X is independently selected from Cl, Br, MsO, TsO and other leaving functional groups.

A treatment method uses a drug containing a hypoxia activated compound of formula (I) in combination therapy with a PARP inhibitor to treat a PARP inhibitor-resistant cancer or tumor patient:

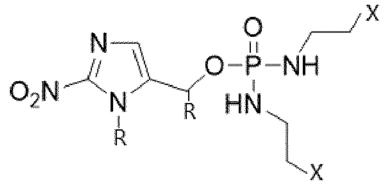

(I),

where each R is independently selected from H, $-CH_3$ and $-CH_2CH_3$, and each X is independently selected from Cl, Br, MsO, TsO and other leaving functional groups.

In the context herein, the "drug" refers to a medicament or formulation. The medicament is prepared so as to contain a hypoxia activated compound of formula (I), or a salt or solvate thereof, as an active ingredient, within a particular dose range, and/or the drug is prepared in a particular dosage form, or for a particular mode of administration.

The medicament, drug or formulation may also be prepared so as to contain a pharmaceutically acceptable adjuvant or excipient. The drug may be in any dosage form for clinical administration, such as tablets, suppositories, dispersible tablets, enteric-coated tablets, chews, orally disintegrating tablets, capsules, dragees, granules, dry powders, oral solutions, solutions for injection in vials or pre-filled syringes, lyophilized powders for injection or infusion solutions. Depending on the dosage form and mode of administration of the drug, the pharmaceutically acceptable adjuvant or excipient may include one or more of a diluent, a solubilizing agent, a disintegrator, a suspending agent, a lubricant, a binding agent, filler, a flavouring agent, a sweetener, an antioxidant, a surfactant, a preservative, a coating agent, a coloring agent and the like.

Formulations related to TH-302 or its analog compound

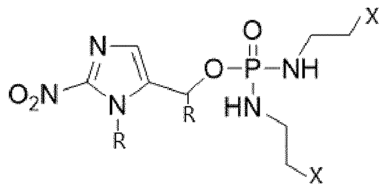

include oral formulations, lyophilized formulations and concentrated injectable solutions, and related regimens, methods of preparation, clinical compatibility and modes of administration have been detailed and disclosed in the following related patent applications filed by Threshold: WO2010048330A1, WO2012142520A2 and WO2008083101A1, the entirety of which is hereby incorporated by reference.

TH-302 or its analog compound

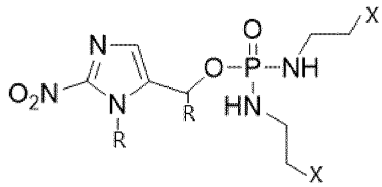

is a DNA-alkylating anti-cancer drug with an extensive cancer treatment potential. Experiments on such related cancer indications and clinical trials have been disclosed in related patent applications filed by Threshold and other pharmaceutical companies (e.g., WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2), as well as in FDA-registered clinical trials (NCT02402062, NCT02020226, NCT02076230, NCT01381822, NCT02093962,

NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567). The entirety of the foregoing related applications and clinical trial information is hereby incorporated by reference.

"Cancer" refers to leukemias, lymphomas, cancers and other malignant tumors (including solid tumors) of potentially unlimited growth, which can expand locally by invasion and systemically by metastasis.

Examples of cancers that can be treated with TH-302 or its anaglog compound

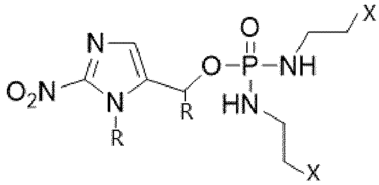,

as listed herein, include (but are not limited to) cancer of the adrenal gland, bone, brain, breast, bronchi, colon and/or rectum, gallbladder, head and neck, kidneys, larynx, liver, lung, neural tissue, pancreas, prostate, parathyroid, skin, stomach, and thyroid. Some other examples of cancers include, acute and chronic lymphocytic and granulocytic tumors, adenocarcinoma, adenoma, basal cell carcinoma, cervical dysplasia and in situ carcinoma, Ewing's sarcoma, epidermoid carcinomas, giant cell tumor, glioblastoma multiforme, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myeloma, mycosis fungoides, neuroblastoma, osteosarcoma, osteogenic and other sarcoma, ovarian tumor, pheochromocytoma, polycythemia vera, primary brain tumor, small-cell lung tumor, squamous cell carcinoma of both ulcerating and papillary type, hyperplasia, seminoma, soft tissue sarcoma, retinoblastoma, rhabdomyosarcoma, renal cell tumor, topical skin lesion, reticulum cell sarcoma and Wilm's tumor.

PARP is an enzyme, fully named as "poly (ADP-ribose) polymerase". PARP is a DNA repair enzyme that plays a crucial role in DNA repair pathways. PARP is activated in response to DNA damage or breaks. As a molecular sensor for DNA damage, it has the function to identify and bind to DNA breaks, thus activating and catalyzing poly-ADP-ribosylation of the receptor protein, which is involved in DNA repair.

A PARP inhibitor can disrupt the normal function of the PARP enzyme that behaves like a "repairer" by inhibiting its action. Without such repair, DNA damaged cells will die.

As PARP is not the only "repairer" in cells, even when PARP fails to work properly, cellular DNA damage will pass down to the next process stage, where there is another "repairer" waiting to repair DNA damage. Proteins produced by the BRCA genes are just important members of the other "repairer". This double security mechanism of normal cells guarantees their survival when one of the security measures fails under the action of a PARP inhibitor and the other still works.

However, in BRCA gene-mutated ovarian cancer or breast cancer cells, the BRCA "repairer" fails to work properly. Of course, as the PARP team still works, the cancer cells will not die.

If a PARP inhibitor specifically enters the cancer cells and disrupts the function of the PARP enzyme therein by inhibiting its activity, DNA damage in the cancer cells cannot be repaired any longer. Thus, the PARP inhibitor will only kill the cancer cells, but not normal cells.

Co-presence of a PARP inhibitor and a BRCA genetic mutation induces so-called "synthetic lethality". Synthetic lethality refers to cellular death caused by variation of both, but not either, of two different genes (BRCA) or proteins (PRAP).

A PARP inhibitor is a compound that has an inhibitory effect on the PARP enzyme. That is, any substance that can inhibit activity of the PARP enzyme is considered as a PARP inhibitor.

The PARP inhibitor is selected from the five commercially available drugs, olaparib, rucaparib, niraparib, talazoparib and fluzoparib, the drug pamiparib that has entered a Phase III clinical trial. Apparently, here, the "PARP inhibitor" essentially refers to a drug containing a PARP inhibitor as an active ingredient.

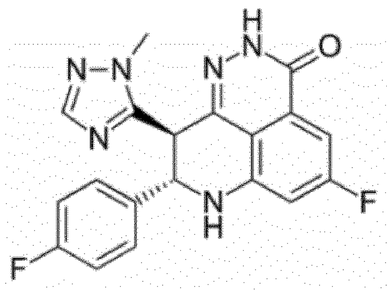

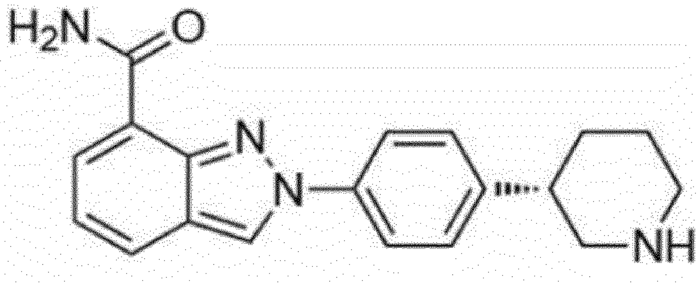

Talazoparib

Niraparib

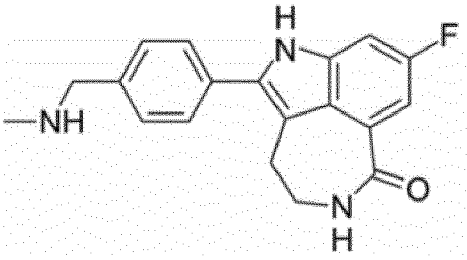

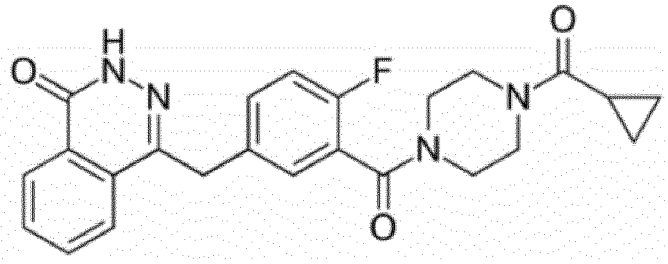

Rucaparib

Olaparib

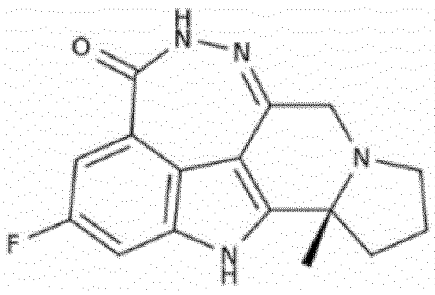

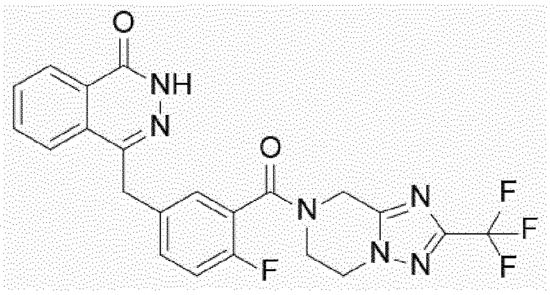

Pamiparib

Fluzoparib

Talazoparib is indicated for the treatment of adult patients with deleterious or suspected deleterious germline BRCA-mutated (gBRCAm) HER2-negative locally advanced or metastatic breast cancer. Commercially available are 0.25 mg/1 mg talazoparib tosylate capsules, and the recommended dose is 1 mg taken orally once daily. For adverse reactions, interruption of treatment or dose reduction can be considered:

After experiencing a first adverse reaction, the oral dose may be reduced to 0.75 mg (three 0.25 mg capsules) once daily;

After experiencing a second adverse reaction, the oral dose may be reduced to 0.5 mg (two 0.25 mg capsules) once daily;

After experiencing a third adverse reaction, the oral dose may be reduced to 0.25 mg (one 0.25 mg capsule) once daily.

Niraparib is indicated for the maintenance treatment of adult patients with platinum-sensitive recurrent epithelial ovarian, fallopian tube or primary peritoneal cancer who are in a complete or partial response to platinum-based chemotherapy. Commercially available are 100 mg niraparib tosylate capsules, and the recommended dose is 300 mg taken orally once daily. Treatment is continued until disease progression or an untolerable adverse reaction. For adverse reactions, interruption of treatment or dose reduction can be considered.

An initial dose reduction may be from three capsules (300 mg) daily to two capsules (200 mg) daily.

If a further dose reduction is required, the dose may be reduced for the second time from two capsules (200 mg) daily to one capsule (100 mg) daily.

If adverse reactions cannot be managed by interruption or dose reduction, discontinuation is recommended.

Rucaparib is indicated for the treatment of women with advanced ovarian cancer and with tumor carrying a particular genetic mutation (deleterious BRCA) who have been treated with two or more chemotherapy drugs. Commercially available are 200 mg, 250 mg and 300 mg tablets. The recommended dose is 600 mg taken orally twice daily with or without food. Treatment is continued until disease progression or unacceptable toxicity. For adverse reactions, interruption of treatment or dose reduction can be considered.

Olaparib is indicated: for the maintenance treatment of treatment-naive adult patients with germline or somatic BRCA-mutated (gBRCAm or sBRCAm) advanced epithelial ovarian, fallopian tube or primary peritoneal cancer who are in complete or partial response to platinum-based chemotherapy; and for the maintenance treatment of adult patients with platinum-sensitive recurrent epithelial ovarian, fallopian tube or primary peritoneal cancer who are in complete or partial response to platinum-based chemotherapy. Commercially available are 150 mg and 100 mg tablets. The recommended dose is 300 mg (two 150 mg tablets) taken twice daily, which is equivalent to a total daily dose of 600 mg. The 100 mg tablets are used for dose reduction.

To manage adverse events such as nausea, vomiting, diarrhea, anemia, etc., interruption of treatment or dose reduction can be considered.

If a dose reduction is required, the recommended dose may be reduced to 250 mg (one 150mg tablet and one 100 mg tablet) taken twice daily (equivalent to a total daily dose of 500 mg).

If a further dose reduction is required, then the recommended dose may be reduced to 200 mg (two 100 mg tablets) taken twice daily (equivalent to a total daily dose of 400 mg).

Fluzoparib is indicated for the treatment of patients with germline BRCA-mutated (gBRCAm) platinum-sensitive recurrent ovarian, fallopian tube or primary peritoneal cancer who have received second- or subsequent-line chemotherapy. Commercially available are 50 mg capsules.

For other candidate PARPi drugs under development, which have entered clinical trials, reference can be made to the web page https://www.selleckchem.com/PARP.html and relevant academic review literature.

For doses of TH-302 or its analog compound

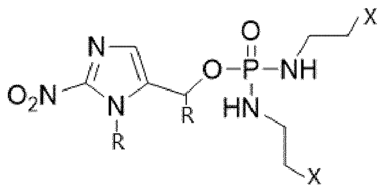

recommended for cancer treatment, reference can be made to the doses described in related patent applications filed by Threshold and other pharmaceutical companies (e.g., WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and FDA-registered clinical trials (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567):

120 mg/m$^2$ to 460 mg/m$^2$ administered daily by intravenous injection;

480 mg/m$^2$ to about 670 mg/m$^2$, or, for example, 575 mg/m$^2$ administered weekly by intravenous injection.

TH-302 used in the clinical trial (a concentrate for preparing an administrable solution) was in the form of a sterile liquid TH-302 preparation. TH-302 was formulated with anhydrous ethanol (70%), dimethylacetamide (25%) and polysorbate 80 (5%). It was provided by the sponsor in rubber-stoppered 10-mL glass vials sealed with flip-off seals. The TH-302 drug product was a clear, colorless to pale yellow solution substantially absent of visible particles. For a nominal total weight of 650 mg of TH-302, each vial for single use contained a nominal filling volume of 6.5 mL of the TH-302 drug product (equivalent to 100 mg/mL) and was clearly labeled with the batch number, route of administration, required storage conditions, name of the sponsor and appropriate warning marks required by the applicable provisions.

Before administration, dilution was conducted according to the pharmacy manual.

Before administration, dilution was conducted with a commercially available 5% aqueous glucose solution to a total volume of 500 mL (1000 mL for a total dose ≥1000 mg) for administration, giving the required final concentration. Each dose of TH-302 was prepared with a di(2-ethylhexyl)phthalate (DEHP)-free 5% aqueous glucose solution and administered as an intravenous drip using a DEHP-free device for administration by intravenous infusion.

Of course, it is also possible to use the lyophilized formulation developed by Threshold:

A solution (20 mL) of TH-302 (100 mg) and sucrose (1 g) was added to a lyophilization vial and lyophilized to yield a lyophilized unit dose form of TH-302 with a drug load of less than 5 mg/$cm^3$. For the purpose of human administration, the unit dose form was dissolved in a 5% glucose injection solution, and an appropriate amount of this solution was administered to patients.

In the subsequent Phase I clinical trial of TH-302 in human patients, the lyophilized formulation was used. The lyophilized TH-302 formulation was prepared for injection in a 100-mL glass vial with a drug load of 100 mg/100 ml and stored at 2-8 °C under controlled conditions. When used, 250mL of a 5% glucose injection solution was injected into the lyophilized formulation vial, and the formulation was administered as an intravenous drip within 30 minutes using an infusion pump.

"Monotherapy" is also known as "single-drug treatment". "Combination therapy" is also known as "combination drug treatment". "Single-drug treatment" refers to using only one anti-cancer drug in one course of treatment. "Combination drug treatment" refers to simultaneously or successively using two or more anti-cancer drugs in one course of treatment.

In general, for combination therapy, it is necessary to attempt various doses and periods of administration according to the characteristics of the condition to be treated and the drugs to be used in combination. A combination therapy regimen can achieve better therapeutic outcomes than monotherapy only if it has been obtained from attempts made according to the aforementioned factors.

Doses and periods of administration for monotherapy and combination therapy regimens should be determined by clinical trials made with reference to the aforementioned doses and dosage regimens for TH-302, the analog compound thereof and the PARPi.

Further, a DNA repair enzyme in the patient is impaired.

According to relevant research literature, the impairment of the DNA repair enzyme is selected from one or more of:

impairment of a homologous recombination DNA repair enzyme;

impairment of a nucleotide excision repair enzyme;

impairment of a non-homologous end-joining enzyme;

impairment of a base excision repair enzyme;

impairment of a mismatch repair enzyme; and

impairment of an enzyme for repair of the Fanconi's anemia pathway.

Preferably, it is any one or more of impairment of a homologous recombination DNA repair enzyme, impairment of a nucleotide excision repair enzyme and impairment of a base excision repair enzyme. More preferably, it is impairment of a homologous recombination DNA repair enzyme alone, or impairment of both a homologous recombination DNA repair enzyme and a nucleotide excision repair enzyme.

Further, any one or two of the genes corresponding to BRCA1/BRCA2 have been determined to be mutated based on the detection of the tumor or cancer tissue of the patient. Alternatively, any one or two of the genes corresponding to BRCA1/BRCA2 have been determined to be mutated based on the detection of the patient.

Any one genetic mutation or two genetic mutations in the genes corresponding to BRCA1/BRCA2 may be detected with commercially available (companion) diagnostic kits:

The olaparib companion test kit, BRACAnalysis CDx, is used to perform genetic testing for identifying BRCA genetic mutations in a blood sample from an ovarian cancer patient.

BRCA1/2 genetic mutation test kits (based on combinatorial probe anchor polymerization sequencing) are used for qualitative testing of germline mutations in exonic and adjacent intronic regions of the BRCA1/2 gene of patients clinically diagnosed with ovarian cancer and breast cancer.

Human BRCA1 and BRCA2 genetic mutation test kits (based on reversible terminator sequencing) can be used to provide related guidance on the usage of olaparib tablets as a PARP inhibitor.

The BRCA1/BRCA2 mutation(s) include(s) germline (gBRCAm) and somatic (sBRCAm) BRCA1/BRCA2 mutation(s).

The hypoxia activated compound of formula (I) is selected from compounds of the following structures:

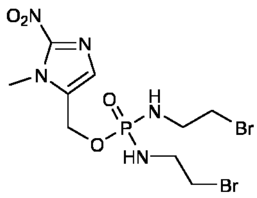

(i.e., TH-302), as well as

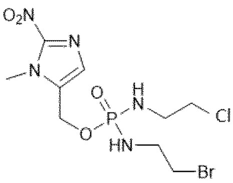 and 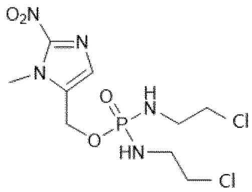 ,

with TH-302 being particularly preferred.

Further, the cancer or tumor is selected from ovarian cancer, breast cancer, pancreatic cancer, fallopian tube cancer, primary peritoneal cancer, gastric cancer, prostate cancer, lung cancer, liver cancer, colon cancer, rectal cancer, bladder cancer, etc., and the lung cancer is preferred to be non-small-cell lung cancer or small-cell lung cancer.

A treatment method uses a drug containing a hypoxia activated compound of the following formula as a monotherapy to treat an olaparib-resistant patient with ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer or bladder cancer:

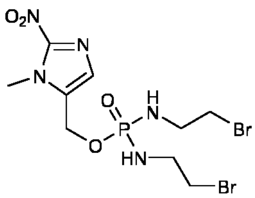 ,

wherein any one or two of the genes corresponding to BRCA1/BRCA2 have been determined to be mutated based on the detection of the tumor or cancer tissue of the patient; or any one or two of the genes corresponding to BRCA1/BRCA2 have been determined to be mutated based on the detection of the patient.

A treatment method uses a drug containing a hypoxia activated compound of the following formula in combination therapy with olaparib to treat an olaparib-resistant patient with ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer or bladder cancer:

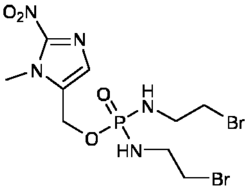 ,

wherein any one or two of the genes corresponding to BRCA1/BRCA2 have been determined to be mutated based on the detection of the tumor or cancer tissue of the patientt; orany one or two of the genes corresponding to BRCA1/BRCA2 have been determined to be mutated based on the detection of the patient.

A treatment method comprises the steps of:

detecting a BRCA1/BRCA2 genetic mutation condition of a PARP inhibitor-resistant cancer or tumor patient;

if there is a BRCA1/BRCA2 genetic mutation in the patient, using a drug containing a hypoxia activated compound of formula (I) as a monotherapy or in combination therapy with a PARP inhibitor for treatment:

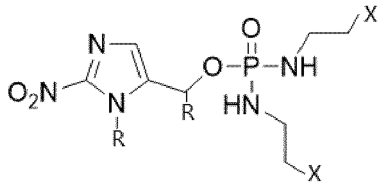

(I),

where each R is independently selected from H, $-CH_3$ and $-CH_2CH_3$, and each X is independently selected from Cl, Br, MsO, TsO and other leaving functional groups.

Preferably, the genetic mutation(s) has(ve) a medium tumor mutational burden (TMB) level.

Use of a hypoxia activated compound of formula (I) for preparing a drug for treating cancer in a patient as a monotherapy or in combination therapy with a PARP inhibitor is provided:

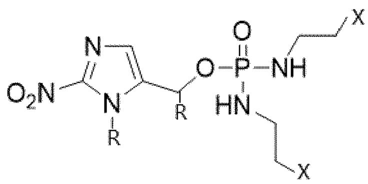

(I),

wherein the patient is a PARP inhibitor-resistant patient; and

each R is independently selected from H, $-CH_3$ and $-CH_2CH_3$, and each X is independently selected from Cl, Br, MsO, TsO and other leaving functional groups.

In the above drug preparation use, a DNA repair enzyme in the patient is impaired; or

any one or two of the genes corresponding to BRCA1/BRCA2 have been determined to be mutated based on the detection of the tumor or cancer tissue of the patient; or

any one or two of the genes corresponding to BRCA1/BRCA2 have been determined to be mutated based on the detection of the patient.

The BRCA1/BRCA2 mutation(s) include(s) germline (gBRCAm) and somatic (sBRCAm) BRCA1/BRCA2 mutation(s).

In the above drug preparation use, the hypoxia activated compound of formula (I) is selected from a compound of the following structure:

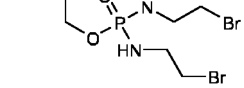

;

or

the PARP inhibitor is selected from olaparib, rucaparib, niraparib, talazoparib, fluzoparib and pamiparib; or

the cancer or tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer and bladder cancer; or

the genetic mutation(s) has(ve) a medium tumor mutational burden (TMB) level.

Different tumors have different tumor mutational burden (TMB) or load values. It is generally believed that a TMB level exceeding 20 mutations/Mb (Mb stands for "per million bases") is high, a TMB level lower than 10 mutations/Mb is low, and a TMB level between the two values is medium. At the World Conference on Lung Cancer 2017, Bristol-Myers Squibb (BMS) disclosed the results from a clinical trial called CheckMate 032. This was a Phase II clinical trial that enrolled 401 patients with advanced lung cancer who had failed first-line treatment, in which they received treatment with a PD-1 inhibitor alone, or a combination of a PD-1 inhibitor and ipilimumab. The patients were divided into three classes: TMB-high, TMB-medium and TMB-low, according to their TMB levels. Among the population who received combination therapy, response rates of the three groups were respectively 62%, 20% and 23%, and the TMB-high population showed a three times higher response rate. Median overall survival periods of the three groups were respectively 22.0 months, 3.6 months and 3.4 months - there was a six times difference between 22.0 months and 3.4 months! This trial demonstrated that, for different cancer-treating drugs, different TMB levels could have a great impact on their efficacy.

The present invention also provides a drug for treating a PARP inhibitor-resistant cancer or tumor patient, which contains a hypoxia activated compound of formula (I) and can be used as a monotherapy or in combination therapy to treat the PARP inhibitor-resistant cancer or tumor patient:

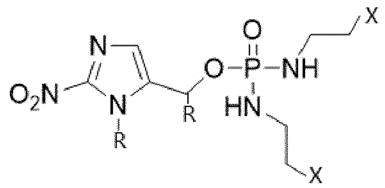

(I),

where each R is independently selected from H, $-CH_3$ and $-CH_2CH_3$, and each X is independently selected from Cl, Br, MsO, TsO and other leaving functional groups.

Preferably, a DNA repair enzyme in the patient is impaired; or

any one or two of the genes corresponding to BRCA1/BRCA2 have been determined to be mutated based on the detection of the tumor or cancer tissue of the patient; or

any one or two of the genes corresponding to BRCA1/BRCA2 have been determined to be mutated based on the detection of the patient.

Preferably, the BRCA1/BRCA2 mutation(s) include(s) germline (gBRCAm) and somatic (sBRCAm) BRCA1/BRCA2 mutation(s).

In particular, in the drug, the hypoxia activated compound of formula (I) is selected from a compound of the following structure:

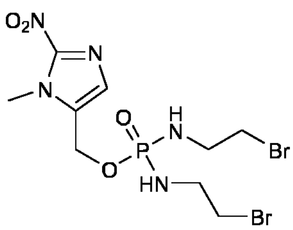

;

or

the PARP inhibitor is selected from olaparib, rucaparib, niraparib, talazoparib, fluzoparib and pamiparib; or

the cancer or tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer and bladder cancer; or

the genetic mutation(s) has(ve) a medium tumor mutational burden (TMB) level.

In addition to the hypoxia activated compound of formula (I), a pharmaceutically acceptable adjuvant or excipient should be added to the drug, depending on the properties of the medicament, drug or formulation. The drug may be in any dosage form for clinical administration, such as tablets, suppositories, dispersible tablets, enteric-coated tablets, chews, orally disintegrating tablets, capsules, dragees, granules, dry powders, oral solutions, solutions for injection in vials or pre-filled syringes, lyophilized powders for injection or infusion solutions. Depending on the dosage form and mode of administration, the pharmaceutically acceptable adjuvant or excipient in the drug may include one or more of a diluent, a solubilizing agent, a disintegrator, a suspending agent, a lubricant, a binding agent, filler, a flavouring agent, a sweetener, an antioxidant, a surfactant, a preservative, a coating agent, a coloring agent and the like.

The drug may be used as a monotherapy or in combination therapy with a PARPi drug.

## BRIEF DESCRIPTION OF THE DRAWINGS

Fig. 1 is a graph showing curves of inhibition rates of the compounds TH-302 and tirapazamine against the Capan-1 cell line under normoxia and hypoxia, in which "con.Log (nM)" represents the logarithms of numerical values of concentrations measured in nmol/L to the base 10, and "inhibition" denotes the inhibition rates.

Fig. 2 is a graph showing curves of inhibition rates of the compounds TH-302 and tirapazamine against the Capan-1 cell line under normoxia and hypoxia, in which "con.Log (nM)" represents the logarithms of numerical values of concentrations measured in nmol/L to the base 10, and "inhibition" denotes the inhibition rates.

Fig. 3 is a graph showing tumor volume growth curves of groups of mice in the human pancreatic cancer Capan-1 subcutaneous model.

Fig. 4 is a graph showing curves of relative tumor growth inhibition rates of groups of mice in the human pancreatic cancer Capan-1 subcutaneous model.

Fig. 5 is a graph showing body weight curves of groups of mice in the human pancreatic cancer Capan-1 subcutaneous model.

Fig. 6 is a graph showing curves of body weight change percentages of groups of mice in the human pancreatic cancer Capan-1 subcutaneous model.

Fig. 7 is a graph showing tumor volume growth curves of groups of mice in the pancreatic cancer Capan-1 CDX model.

Fig. 8 is a graph showing curves of body weights of treatment groups in the pancreatic cancer Capan-1 CDX model varying over time.

Fig. 9 is a graph showing tumor volume growth curves of groups of mice in the lung cancer LU6429 PDX model.

Fig. 10 is a graph showing curves of body weights of treatment groups in the lung cancer LU6429 PDX model varying over time.

Fig. 11 is a graph showing tumor volume growth curves of groups of mice in the bladder cancer BL3325 PDX model.

Fig. 12 is a graph showing curves of body weights of treatment groups in the bladder cancer BL3325 PDX model varying over time.

## DETAILED DESCRIPTION

The present invention will be described below with respect to specific examples. Those skilled in the art will understand that these examples are only intended to illustrate the present invention and are not intended to limit the scope thereof in any sense.

All the experimental methods used in the following examples are regular methods unless particularly specified. All the pharmaceutical raw materials, reagents, materials and the like are commercially available products, unless particularly specified.

The terms "patient" and "individual" are interchangeable and refer to a mammal in need of treatment for cancer. Generally, the patient is a human. Generally, the patient is a human diagnosed with cancer. In some embodiments, a "patient" or "individual" may be a non-human mammal used in screening, characterizing, and evaluating drugs and therapies, such as a non-human primate, a dog, cat, rabbit, pig, mouse or rat.

"Prodrug" refers to a compound that, after dosage or administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is dosed. A prodrug may have, relative to the active drug, altered metabolic stability or transport characteristics, fewer side effects or lower toxicity, or improved flavor. A prodrug may be synthesized using reactants other than the corresponding drug.

"Treating" or "treating a patient" refers to dosing, applying or administering to the patient a therapeutically effective amount of a drug that the present invention is directed to.

"Dosing", "administering" or "applying" a drug to a patient refers to direct dosage or administration (which may be dosage or administration to a patient by a medical professional, or may be self-dosage or self-administration) and/or indirect dosage or administration, which may be the act of prescribing a drug. For example, a physician who instructs a patient to self-deliver or self-administer a drug and/or provides a patient with a prescription for a drug is, delivering or administering the drug to the patient.

"Therapeutically effective amount" of a drug refers to an amount of a drug that, when dosed, administered or applied to a patient with cancer, will have the intended therapeutic effect (e.g., alleviation, amelioration, palliation or elimination of one or more clinical manifestations of cancer in the patient). A therapeutic effect does not necessarily occur by dosage or administration of one dose, and may occur only after dosage or administration of a series of doses. Thus, a therapeutically effective amount may be dosed or administered in one or more dosages or administrations.

"Treatment" of a condition or patient refers to taking steps to obtain beneficial or desired results (including clinical results). For purposes of this invention, beneficial or desired clinical results include (but are not limited to) alleviation or amelioration of one or more symptoms of cancer; diminishment of extent of disease; delay or slowing of disease progression; amelioration, palliation, or stabilization of the disease state; or other beneficial results. In some instances, cancer treatment may result in a partial response or disease stabilization.

"Tumor cells" refers to tumor cells of any suitable species (e.g., a mammal, such as a rodent, a dog, a cat, a horse or a human).

The foregoing description of specific embodiments of the present invention is not intended to limit the invention, and those skilled in the art can make various modifications or variations based on the teachings herein. Such modifications or variations, as long as they do not depart from the spirit of the present invention, are all intended to fall within the scope of the invention as defined by the appended claims.

Specific experiments performed in accordance with the present invention are set forth below.

The protocols of animal experiments performed in tests disclosed herein, any modifications thereof, and the welfare and use of animals were reviewed and approved by the IACUC committee of the applicable Contract Research Organization (CRO). During the tests, animal welfare and experimental operations were compliant with the AAALAC requirements.

### 1. Comparison of In Vitro Cytotoxicity of Compound TH-302 towards BRCA-Knockout and Wild-Type Tumor Cells

TH-302 is a small-molecule prodrug that can be activated under hypoxia to release cytotoxin, thereby killing tumor cells and damaging tumor tissue. In order to evaluate the correlation between TH-302 and pathogenic BRCA mutations on the level of in vitro cells, we chose the human colon cancer cell line DLD1 and a DLD1-BRCA2$^{-/-}$ tumor cell line with the BRCA2 protein being knocked out and verified whether TH-302 exhibited a difference in the ability to kill the two tumor cell lines with and without BRCA2 protein expression under hypoxia.

In this clonogenic assay, $IC_{90}$ (90% inhibitory concentration) values were used to evaluate the cell-killing ability of the compound TH-302. The detailed experimental method was as follows:

(1) Cell Culture

a) The DLD1 and DLD1-BRCA2$^{-/-}$ cells were cultured in RPMI medium supplemented with 10% FBS and 1% Anti/Anti and incubated at 37 °C in 5% $CO_2$.

(2) Cell Seeding

a) The cells were regularly incubated to 80-90% confluence, and were harvested when desired numbers were reached.

b) The cells were resuspended in corresponding medium, counted and prepared into cell suspensions with proper densities.

c) The cell suspensions were added to 10-cm glass dishes. The DLD1-BRCA2$^{-/-}$ cells were present at a density of $3.0 \times 10^5$ per dish, and the DLD1 cells were present at a density of 300 per dish.

d) The cells were incubated in an incubator at 37 °C in 5% $CO_2$ for two days.

(3) Preparation of Compound
The compound was prepared, as required by the experiment.

(4) Treatment of Cells with Compound

a) The cells were treated for 3 hours with the compound under a condition with an oxygen content <0.01% (hypoxic).

b) The cells were washed once with $1 \times$ PBS, digested with pancreatin and counted.

c) The cells were resuspended in 3 mL of medium and inoculated into 6-well culture plates. The DLD1-BRCA2$^{-/-}$ cells were present at a density of 2000 per well, and the DLD1 cells were present at a density of 300 per well.

d) The plates with the cells were placed in an incubator for 10 days.

e) The medium was discarded, and the cells were fixed and stained with crystal violet for 40 minutes.

f) A colony counter (VWR) was used to count colonies of the cells, and $IC_{90}$ values were calculated using the software CalcuSyn (http://www.biosoft.com/w/calcusyn.htm).

The $IC_{90}$ values of the compound TH-302 under test against the two types of cells, as measured by the above method, are listed in Table 1 blow.

Table 1 $IC_{90}$ Data Showing Inhibitory Effects of Compound TH-302 on Two Tumor Cell Strains under Hypoxia

| Compound | Cell Line | $IC_{90}$ (nmol/L) | Difference (times) |
|---|---|---|---|
| TH-302 | DLD1 | 210 | / |
| | DLD1-BRCA2$^{-/-}$ | 3.01 | 70 |

Experimental conclusions: after being activated under hypoxia, the compound TH-302 showed a significant difference in in vitro cytotoxicity to the wild-type DLD1 cells and BRCA2-deficient DLD1-BRCA2-/- cells. Compared with the DLD1-BRCA2$^{-/-}$ cells, the in vitro $IC_{90}$ value of the wild-type DLD1 cells increased by 70 times. These results demonstrate that the deficiency of the BRCA2 protein significantly enhances sensitivity of the DLD1 cells to TH-302 under hypoxia.

**2. Experiment on Impact of Compound TH-302/Tirapazamine on In Vitro Cell Proliferation of Capan-1/Bxpc-3**

The applicant specially studied in vitro cell proliferation inhibition of Capan-1 and BxPc-3 cell lines by the

hypoxia-activated anti-cancer prodrugs TH-302 and tirapazamine under normoxia and hypoxia. The Capan-1 cell line was a BRCA-mutated cell line, and the BxPc-3 cell line was a BRCA wild-type (i.e., non-BRCA-mutated) cell line. This experiment was intended to verify the difference of the hypoxia-activated anti-cancer prodrugs, TH-302 and tirapazamine, in sensitivity to BRCA mutations.

The method, data and conclusions of the experiment are set forth below.

**Experimental Method**

1) Capan-1/BxPc-3 cell suspensions were added to two types of 24-well plates, 495 μL each well. Density of the cells was $6\times10^4$ per well. 24-well plates with glass inserts were used in hypoxic tests, and ordinary plastic 24-well plates were used in normoxic tests.

2) The cells were incubated overnight in an incubator at 37 °C in 5% $CO_2$.

3) Compound Processing

Hypoxia:

A hypoxia workstation was tuned to create a hypoxic environment ($O_2$<0.01%), and an oxygen indicator was used to confirm the hypoxia in the workstation.

24 hours after cell seeding, the 24-well plates with glass inserts were transferred into the hypoxia workstation.

The 24-well plates were placed on vortex shakers, uncovered and shaken to allow air exchange for 5 minutes.

To each well in a monotherapy group, a 100× test compound solution was added.

Normoxia:

24 hours after cell seeding, 5 μL of the 100× test compound solution was added to each well. Three replicate wells were set up for each test group.

To each well in the monotherapy group, the 100× test compound solution was added.

4) 3 hours after compound processing, all the 24-well plates were washed twice with complete medium, 500 μL for each well every time.

5) Each well in the Capan-1 cell culture plates was added with 1000 μL of complete medium, and each well in the BxPc-3 cell culture plates was added with 500 μL of complete medium.

6) They were placed in an incubator for 72 hours at 37 °C in 5% $CO_2$.

7) 800 μL of medium in each Capan-1 cell culture well and 300 μL of medium in each BxPc-3 cell culture well was discarded, and 50 μL of CTG was added, followed by shaking for 2 minutes to homogeneity and placement for 15 minutes at room temperature without exposure to light.

8) 100 μL of medium was transferred from each well of the 24-well plates into 96-well blank plates.

9) A multimode plate reader was used to read values of chemiluminescence signals for 1000 ms.

10) $IC_{50}$ calculations were performed using the software GraphPad Prism 5, obtaining $IC_{50}$ (half-inhibitory concentration) values of the compounds.

**Experimental Data**

Data of the experiment on cell proliferation inhibition of the BRCA-mutated Capan-1 cell line by TH-302 and tirapazamine under normoxia and hypoxia is presented in Tables 2 and 3, and the $IC_{50}$ curves are shown in Fig. 1.

Table 2 Data of Experiment on Cell Proliferation Inhibition of BRCA-Mutated Capan-1 Cell Line by TH-302

| Compound | $IC_{50}$ (μM) | Ratio (21% $O_2$/0.01% $O_2$) |
|---|---|---|
| TH-302, 21% $O_2$ | 93.45 | - |
| TH-302, <0.01% $O_2$ | 0.82 | 113.96 |

Table 3 Data of Experiment on Cell Proliferation Inhibition of BRCA-Mutated Capan-1 Cell Line by Tirapazamine

| Compound | $IC_{50}$ (μM) | Ratio (21% $O_2$/0.01% $O_2$) |
|---|---|---|
| tirapazamine, 21% $O_2$ | >2000 | - |
| tirapazamine, <0.01% $O_2$ | 29.06 | >68.82 |

Data of the experiment on cell proliferation inhibition of the BRCA wild-type BxPc-3 cell line by TH-302 and tirapazamine under normoxia and hypoxia is presented in Tables 4 and 5, and the $IC_{50}$ curves are shown in Fig. 2.

Table 4 Data of Experiment on Cell Proliferation Inhibition of BRCA Wild-Type BxPc-3 Cell Line by TH-302

| Compound | $IC_{50}$ (μM) | Ratio (21% $O_2$/0.01% $O_2$) |
|---|---|---|
| TH-302, 21% $O_2$ | 423.50 | - |
| TH-302, <0.01% $O_2$ | 3.07 | 137.95 |

Table 5 Data of Experiment on Cell Proliferation Inhibition of BRCA Wild-Type BxPc-3 Cell Line by Tirapazamine

| Compound | $IC_{50}$ (μM) | Ratio (21 % $O_2$/0.01 % $O_2$) |
|---|---|---|
| tirapazamine, 21% $O_2$ | >2000 | - |
| tirapazamine, <0.01% $O_2$ | 33.23 | >60.19 |

**Experimental Conclusions**

In the above experimental data, $IC_{50}$ of TH-302 under hypoxia was 0.82 μM for the BRCA-mutated Capan-1 cell line, and was 3.07 μM for the BRCA wild-type BxPc-3 cell line, and there was a 3.7 times difference between the two values. This indicates that BRCA mutations induce stronger proliferation inhibition activity of TH-302 against a tumor cell line. That is, BRCA mutations enhance sensitivity of tumor cells to the drug TH-302.

In contrast, $IC_{50}$ of the other hypoxia-activated anti-cancer prodrug, tirapazamine, under hypoxia was 29.06 μM for the BRCA-mutated Capan-1 cell line, and was 33.23 μM for the BRCA wild-type BxPc-3 cell line. The difference was 1.1 times and insignificant. This indicates that tirapazamine is not correlated with BRCA mutations. In other words, BRCA mutations do not have a remarkable impact on the proliferation inhibition activity of tirapazamine against a tumor cell line. That is, BRCA mutations cannot enhance sensitivity of tumor cells to the drug tirapazamine.

**3. Experiments on Efficacy and Safety of TH-302 in Olaparib-Resistant CDX and PDX Animal Models**

**3.1 Evaluation of Efficacy and Safety of TH-302 in Olaparib-Resistant Pancreatic Cancer Capan-1 CDX Model**

The Capan-1 CDX model was an olaparib-resistant model with a pathogenic BRCA2 mutation.

Each nude female BALB/c mouse was subcutaneously inoculated at a lower portion of the right back with $5\times10^5$ Capan-1 cells resuspended in a 1:1 mixture of PBS and matrigel (0.1 ml per mouse). A total of 64 female mice were inoculated. The inoculation was conducted on June 23, 2021. When tumors grew to an average volume of 140 $mm^3$, the mice were randomized by their tumor size into a total of 7 groups, 6 in each group: 100 mg/kg of the test drug olaparib as a monotherapy (Group 2); 75 mg/kg TH-302 in combination therapy with 100 mg/kg olaparib (Group 5); 75 mg/kg TH-302 as a monotherapy (Group 7); and 10% anhydrous ethanol + 10% polyoxyethylene (35) castor oil +80% glucose injection solution D5W (pH 7.4) as a vehicle control. The vehicle control group and the TH-302 monotherapy and combination therapy groups were all administered through tail vein, once weekly for totally three weeks. The test drug olaparib group was orally and intragastrically administered, once daily for a total of 30 days. Efficacy was evaluated based on relative tumor growth inhibition (TGI) rates (%), and safety was evaluated based on body weight changes and deaths of the animals.

No tumor growth inhibition effect was observed in the 100 mg/kg test drug olaparib treatment group (Group 2) on Day 35 after tumor cell inoculation - the relative tumor growth inhibition (TGI) rate (%) was -7.1%, not showing a

statistically significant difference compared with the control group (p>0.05). A significant tumor growth inhibition effect was obtained in the 100 mg/kg olaparib + 75 mg/kg TH-302 combination therapy group (Group 5) on Day 35 after tumor cell inoculation. Compared with the control group, there was a statistically significant difference (p<0.001), and the relative tumor growth inhibition (TGI) rate (%) was 84.47%. A significant tumor growth inhibition effect was obtained in the 75 mg/kg TH-302 monotherapy treatment group (Group 7) on Day 35 after tumor cell inoculation. Compared with the control group, there was a statistically significant difference (p<0.001), and the relative tumor growth inhibition (TGI) rate (%) was 87.66%. No significant difference (p>0.05) in tumor growth inhibition effect was found in a comparison drawn between the TH-302 monotherapy group and the olaparib + TH-302 combination therapy group. No significant body weight losses were observed in the mice of the 100 mg/kg test drug olaparib, 75 mg/kg TH-302 monotherapy and 100 mg/kg olaparib + 75 mg/kg TH-302 combination therapy groups, demonstrating good tolerance.

The dosage regimen of each group is specifically shown in Table 6 below.

Table 6 Drug Dosage Regimens of Groups in Experiment on Human Pancreatic Cancer Capan-1 Subcutaneous Animal Treatment Model

| Group | # of Animals | Treatment Group | Dose (mg/kg) | Route of Administration | Dosage Cycle |
|---|---|---|---|---|---|
| 1 | 6 | 10% anhydrous ethanol + 10% polyoxyethylene (35) castor oil + 80% glucose injection solution D5W (pH 7.4) | - | *i.v.* | QW×3 |
| 2 | 6 | olaparib | 100 | p.o. | QD×30 |
| 5 | 6 | TH-302 | 75 | *i.v.* | QW×3 |
| | | olaparib | 100 | p.o. | QD×30 |
| 7 | 6 | TH-302 | 75 | *i.v.* | QW×3 |

Tumor volumes of the mice in the groups were measured on different days, and average values were calculated. The results are summarized in Table 7 below.

Table 7 Variation of Tumor Volumes of Groups of Mice in Human Pancreatic Cancer Capan-1 Model over Time of Treatment (measured in $mm^3$)

| Test Group and Day # after Inoculation | Group 1 10% anhydrous ethanol +10% polyoxyethylen e (35) castor oil + 80% glucose injection solution D5W (pH 7.4) | Group 2 olaparib, 100 g/kg | Group 5 TH-302, 75 mg/kg, in combination with olaparib, 100 mg/kg | Group 7 TH-302, 75 mg/kg |
|---|---|---|---|---|
| Day 5 after cell inoculation | 140.15±12.35 | 140.69±13.31 | 140.09±11.54 | 141.29±13.45 |
| Day 7 after cell inoculation | 211.60±32.78 | 255.26±56.18 | 243.11±27.79 | 240.00±28.16 |
| Day 9 after cell inoculation | 257.42+44.31 | 288.46+52.25 | 284.67+48.75 | 272.65+29.49 |
| Day 12 after cell inoculation | 333.44+61.23 | 406.96+81.71 | 318.67+64.40 | 280.18+23.72 |
| Day 14 after cell inoculation | 412.38±79.84 | 482.18±88.80 | 305.16±67.67 | 250.13±18.31 |

(continued)

| Test Group and Day # after Inoculation | Group 1 10% anhydrous ethanol +10% polyoxyethylen e (35) castor oil + 80% glucose injection solution D5W (pH 7.4) | Group 2 olaparib, 100 g/kg | Group 5 TH-302, 75 mg/kg, in combination with olaparib, 100 mg/kg | Group 7 TH-302, 75 mg/kg |
|---|---|---|---|---|
| Day 16 after cell inoculation | 433.99±69.21 | 534.69±95.62 | 281.13±69.23 | 216.81±15.63 |
| Day 19 after cell inoculation | 494.74+69.69 | 652.86±114.19 | 250.26±69.38 | 186.76±25.72 |
| Day 21 after cell inoculation | 635.79±77.63 | 716.59±145.84 | 254.94±75.89 | 170.43±16.07 |
| Day 23 after cell inoculation | 689.93±101.71 | 709.38±163.04 | 227.48±80.51 | 162.42±15.97 |
| Day 26 after cell inoculation | 790.81±133.21 | 787.78±164.59 | 213.51±73.21 | 149.66±15.68 |
| Day 28 after cell inoculation | 918.85±158.54 | 870.02±198.09 | 195.29±69.19 | 132.20±12.63 |
| Day 30 after cell inoculation | 999.61±152.57 | 1007.07±217.15 | 195.08±70.44 | 125.55±16.37 |
| Day 33 after cell inoculation | 1104.13±160.17 | 1169.84±201.70 | 189.89±60.89 | 135.67±17.99 |
| Day 35 after cell inoculation | 1175.78±150.36 | 1306.53±210.78 | 203.80±69.82 | 147.81±17.94 |

Tumor growth of the treatment groups and the control group is shown in Table 7 and Fig. 3. Efficacy evaluation is shown in Table 8.

Table 8 Efficacy Analysis of Groups in Human Pancreatic Cancer Capan-1 Subcutaneous Model

| **Test Group** | **Day 35 after Cell Inoculation (07/28/2021)** | | | | |
|---|---|---|---|---|---|
| | **Tumor Volume ($\bar{x}$±S)** | **Relative Tumor Volume ($\bar{x}$±S)** | **TGI (%)** | **T/C (%)** | **P Value (in comparison with control group)** |
| Group 1 10% anhydrous ethanol + 10% polyoxyethylene (35) castor oil + 80% glucose injection solution D5W (pH 7.4) | 1175.78±150. 36 | 8.61±1.08 | - | - | - |
| Group 2 100 mg/kg olaparib | 1306.53±210. 78 | 9.22±1.36 | -7.1 | 107.1 | 1 |
| Group 5 TH-302, 75 mg/kg, in combination with olaparib, 100 mg/kg | 203.80±69.82 | 1.34±0.38 | 84.47 | 15.53 | <0.001 |

(continued)

| Test Group | Day 35 after Cell Inoculation (07/28/2021) | | | | |
|---|---|---|---|---|---|
| | Tumor Volume ($\bar{x}\pm S$) | Relative Tumor Volume ($\bar{x}\pm S$) | TGI (%) | T/C (%) | P Value (in comparison with control group) |
| Group 7 TH-302, 75 mg/kg | 147.81±17.94 | 1.06±0.13 | 87.66 | 12.34 | <0.001 |

In Table 8, T/C (%) represents a relative tumor proliferation rate at a certain point of time, which is calculated as a relative tumor volume or a tumor weight of a treatment group as a percentage of that of the control group according to:

$$T/C\ \% = T_{RTV} / C_{RTV} \times 100\%$$

(where $T_{RTV}$ denotes average RTV of the treatment group; $C_{RTV}$ represents average RTV of the vehicle control group; and RTV=$V_t/V_0$, where $V_0$ is a tumor volume of an animal at the time of randomization, and $V_t$ is a tumor volume of the animal after treatment).

The relative tumor growth inhibition rate, TGI (%), is calculated according to: TGI (%) = (1-T/C) × 100% (where T and C are average relative tumor volumes (RTVs) of a treatment group and the control group, respectively, at a particular point of time).

Table 9 Relative Tumor Growth Inhibition Rates of Groups in Human Pancreatic Cancer Capan-1 Subcutaneous Model

| Group | Days after Inoculation | | | | | | | | | | | | | |
|---|---|---|---|---|---|---|---|---|---|---|---|---|---|---|
| | 6/28/2021 | 6/30/2021 | 7/212021 | 7/5/2021 | 7/7/2021 | 7/9/2021 | 7/12/2021 | 7/14/2021 | 7/16/2021 | 7/19/2021 | 7/21/2021 | 7/23/2021 | 7/26/2021 | 7/28/2021 |
| | | 7 | 9 | 12 | 14 | 16 | 19 | 21 | 23 | 26 | 28 | 30 | 33 | 35 |
| Group 01 | | | | | | | | | | | | | | |
| Group 02 | -0.39% | -20.63% | -12,06% | -22.05% | -16.93% | -23.20% | -31.96% | -12.71% | -2.82% | 0.38% | 5.31% | -0.75% | -5.95% | -11.12% |
| Group 05 | 0.04% | -14.89% | -10.58% | 4.43% | 26.00% | 35.22% | 49.42% | 59.90% | 67.03% | 73.00% | 71.75% | 80.48% | 82.80% | 82.67% |
| Group 07 | -0.82% | -13.42% | -5.91% | 15.97% | 39.34% | 50.04% | 62.25% | 73.19% | 76.46% | 81.08% | 85.61% | 87.44% | 87.71% | 87.43% |

Data in this table is plotted as the curves in Fig. 4.

Body weights of the mice in the groups were measured on different days, and average values were calculated. The results are summarized in Table 10 below.

Table 10 Body Weights of Mice in Human Pancreatic Cancer Capan-1 Subcutaneous Animal Treatment Model after Different Days of Inoculation in Drug Experiment

| Group | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 |
|---|---|---|---|---|---|---|---|---|---|---|---|---|---|---|---|---|---|---|---|---|---|---|---|---|---|---|---|---|---|---|---|
| Group 01 | 22.1 | | 21.5 | | 22.5 | | | 22.7 | | 23.5 | | 23.7 | | | 24.3 | | 24.0 | | 24.2 | | | 25.3 | | 24.7 | | 25.0 | | | 25.4 | | 25.2 |
| Group 02 | 22.8 | 22.5 | 22.6 | 22.7 | 22.6 | 23.0 | 22.7 | 22.7 | 23.3 | 23.2 | 23.2 | 23.3 | 23.1 | 23.6 | 23.5 | 23.3 | 23.4 | 23.6 | 23.8 | 23.8 | 23.7 | 23.9 | 24.0 | 24.0 | 23.7 | 23.9 | 24.5 | 24.3 | 24.4 | 24.3 | 24.5 |
| Group 05 | 22.6 | 22.4 | 22.3 | 22.4 | 22.2 | 22.6 | 22.6 | 22.1 | 22.8 | 23.0 | 22.8 | 22.5 | 22.4 | 22.7 | 22.6 | 22.5 | 22.5 | 22.6 | 22.9 | 22.7 | 22.7 | 23.0 | 23.1 | 23.0 | 23.0 | 23.4 | 23.5 | 23.5 | 23.5 | 23.3 | 23.2 |
| Group 07 | 22.7 | | 22.0 | | 22.7 | | | 23.4 | | 23.6 | | 23.6 | | | 23.8 | | 23.7 | | 23.8 | | | 23.3 | | 24.1 | | 24.3 | | | 24.7 | | 24.7 |

Data in this table is plotted as the curves in Fig. 5, which show body weights of the mice in the human pancreatic cancer Capan-1 subcutaneous model in the groups.

Similarly, Table 11 below can be obtained by processing the data in Table 10.

Table 11 Body Weight Change Percentages of Mice in Human Pancreatic Cancer Capan-1 Subcutaneous Animal Treatment Model after Different Days of Inoculation in Drug Experiment (% Group Mean Change = mean((T-T0)/T0) * 100, wherein T denotes a current value, and T0 represents an initial value)

| Group | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 |
|---|---|---|---|---|---|---|---|---|---|---|---|---|---|---|---|---|---|---|---|---|---|---|---|---|---|---|---|---|---|---|---|
| Group 01 | 0.00 | | -2.43 | | 2.06 | | | 2.98 | | 6.76 | | 7.27 | | | 10.01 | | 8.85 | | 9.92 | | | 14.59 | | 12.07 | | 13.24 | | | 14.93 | | 14.04 |
| Group 02 | 0.00 | -1.18 | -0.91 | -0.35 | -0.82 | 1.04 | -0.25 | -0.61 | 2.18 | 1.87 | 1.86 | 2.16 | 1.43 | 3.45 | 3.00 | 2.19 | 2.68 | 3.51 | 4.38 | 4.47 | 3.80 | 4.97 | 5.39 | 5.29 | 4.05 | 4.85 | 7.24 | 6.56 | 6.92 | 6.71 | 7.26 |
| Group 05 | 0.00 | -0.92 | -1.15 | -0.62 | -1.63 | 0.17 | 0.17 | -1.97 | 0.99 | 2.03 | 1.08 | -0.27 | -0.88 | 0.34 | 0.27 | -0.47 | -0.19 | 0.17 | 1.65 | 0.51 | 0.58 | 1.87 | 2.51 | 1.69 | 2.04 | 3.49 | 4.04 | 4.21 | 3.90 | 3.11 | 2.79 |
| Group 07 | 0.00 | | -3.01 | | 0.18 | | | 2.90 | | 3.78 | | 4.07 | | | 5.01 | | 4.49 | | 4.78 | | | 2.63 | | 6.22 | | 6.96 | | | 8.63 | | 8.65 |

Data in this table is plotted as the curves in Fig. 6.

From analysis of the experimental data, the following findings were noted in terms of efficacy:

1. The Capan-1 CDX model was a true olaparib-resistant model, and olaparib had no inhibitory effect on tumor growth of the model. That is, it possessed resistance to olaparib.

2. TH-302 as a monotherapy exerted a good therapeutic effect on olaparib-resistant pancreatic cancer (TGI=82.67%).

3. TH-302 in combination therapy with olaparib had a good therapeutic effect on olaparib-resistant pancreatic cancer (TGI=87.43%).

4. Compared with the olaparib + TH-302 combination therapy group, the TH-302 monotherapy group achieved slightly better tumor growth inhibition, but there was no significant difference therebetween ($p>0.05$).

Analysis of the experimental data revealed that none of the mice in the 100 mg/kg test drug olaparib, 75 mg/kg TH-302 monotherapy and 100 mg/kg olaparib + 75 mg/kg TH-302 combination therapy groups experienced significant body weight losses, showing good tolerance.

The inventors perform in-depth studies of TH-302 on the olaparib-resistant pancreatic cancer Capan-1 CDX model and found that (1) the TH-302 monotherapy treatment group exhibited a dose-dependent drug tumor growth inhibition effect, and that (2) a particular dose combination of olaparib and TH-302 in combination therapy created a synergistic tumor growth inhibition effect.

The inventors also carried out efficacy and safety studies of TH-302 on the olaparib-resistant lung cancer LU6429 PDX and bladder cancer BL3325 PDX models.

Protocols and experimental data of these in-depth studies are set forth below.

### 3.2 In-Depth Efficacy and Safety Studies of TH-302 on Olaparib-Resistant Pancreatic Cancer Capan-1 CDX Model

Protocol: Nude BALB/c mice were subcutaneously inoculated with human pancreatic cancer Capan-1 cells, establishing a human pancreatic cancer subcutaneous xenograft model. The mice were divided into 7 groups, 6 in each group: 100 mg/kg of the test drug olaparib as a monotherapy (Group 2); 50 mg/kg of TH-302 as a monotherapy (Group 3, QD); 100 mg/kg of TH-302 as a monotherapy (Group 4); 50 mg/kg of TH-302 as a monotherapy (Group 5, QW); 25 mg/kg of TH-302 as a monotherapy (Group 6); 25 mg/kg TH-302 in combination therapy with 100 mg/kg olaparib (Group

7); and 10% anhydrous ethanol + 10% polyoxyethylene (35) castor oil + 80% glucose injection solution D5W (pH 7.4) as a vehicle control (Group 1). The vehicle control group and the TH-302 test groups were all administered through tail vein injection. The 50 mg/kg TH-302 monotherapy group (Group 3, QD) was administered once daily for 3 consecutive days. After rest for 4 days and two weeks, the mice were again administered daily for another 3 consecutive days. TH-302 in all the 100 mg/kg (Group 4, QW), 50 mg/kg (Group 5, QW) and 25 mg/kg (Group 6, QW) TH-302 monotherapy groups and 25 mg/kg TH-302 + 100 mg/kg olaparib combination therapy group (Group 7) was administered once weekly for a total of three weeks. The test drug olaparib in all the groups was orally and intragastrically administered once daily for totally 30 days. Routes of administration, doses and regimens for the human pancreatic cancer Capan-1 animal model are specifically summarized in Table 12.

**Table 12 Routes of Administration, Doses and Regimens for Human Pancreatic Cancer Capan-1 Animal Model**

| Group | # of Animals | Treatment Group | Dose (mg/ kg ) | Route of Administration | Dosage Cycle |
|---|---|---|---|---|---|
| 1 | 6 | 10% anhydrous ethanol + 10% polyoxyethylene (35) castor oil + 80% glucose injection solution D5W (pH 7.4) | - | *i.v.* | QW×3 |
| 2 | 6 | olaparib | 100 | p.o. | QD×30 |
| 3 | 6 | TH-302 | 50 | *i.v.* | QD×3, 4 days off, 2 weeks off; QD×3 |
| 4 | 6 | TH-302 | 100 | *i.v.* | QW×3 |
| 5 | 6 | TH-302 | 50 | *i.v.* | QW×3 |
| 6 | 6 | TH-302 | 25 | *i.v.* | QW×3 |
| 7 | 6 | TH-302 | 25 | *i.v.* | QW×3 |
| | | olaparib | 100 | p.o. | QD×30 |
| Note: Volumes of administration were 10 μL/g. | | | | | |

Tumor growth of the treatment and control groups on different days in the experiment were recorded and shown in Table 13, and corresponding tumor volume growth curves of the mice in respective groups are shown in Fig. 7. Efficacy evaluation was conducted based on relative tumor proliferation rates and relative tumor growth inhibition rates, and the results of efficacy analysis are summarized in Table 14. Body weight changes of the treatment and control groups after administration were recorded to investigate safety of the groups in the human pancreatic cancer Capan-1 subcutaneous model. Body weight change rates of the mice on Day 43 are shown in Table 15, and curves showing body weights of the treatment groups over time are plotted in Fig. 8.

A relative tumor proliferation rate, denoted as T/C %, is calculated as a relative tumor volume or a tumor weight of a treatment group as a percentage of that of the control group at a certain point of time according to:

T/C % = $T_{RTV}$ / $C_{RTV}$ × 100% (where $T_{RTV}$ denotes average RTV of the treatment group; $C_{RTV}$ represents average RTV of the vehicle control group; and RTV=$V_t/V_0$, where $V_0$ is a tumor volume of an animal at the time of randomization, and $V_t$ is a tumor volume of the animal after treatment); or

T/C % = $T_{Tw}$ / $C_{TW}$ × 100% (where $T_{TW}$ is an average tumor weight of the treatment group at the end of the experiment, and $C_{TW}$ is an average tumor weight of the vehicle control group at the end of the experiment).

A relative tumor growth inhibition rate, denoted as TGI (%), is calculated according to: TGI% = (1-T/C) × 100% (where T and C are relative tumor volumes (RTV) or tumor weights (TW) of a treatment group and the control group, respectively, at a particular point of time).

**Table 13 Variation of Tumor Volumes of Groups of Mice in Human Pancreatic Cancer Capan-1 Model over Time of Treatment ($mm^3$)**

| Group | Days after Inoculation | | | | | | | | | | | |
|---|---|---|---|---|---|---|---|---|---|---|---|---|
| | Day 6 | Day 9 | Day 13 | Day 16 | Day 20 | Day 23 | Day 27 | Day 30 | Day 34 | Day37 | Day 41 | Day 43 |
| Group 1 | 146.7 3±13. 49 | 202.65 ±26.64 | 255.59 ±42.25 | 381.63 ±74.7 4 | 415.53 ±69.72 | 458.56 ±73.18 | 574.50 ±78.37 | 698.89 ±86.2 8 | 802.76 ±63.70 | 956.6 0±91. 72 | 1225.5 8±50.1 9 | 1301.3 8±76.6 1 |
| Group 2 | 146.7 2±15. 47 | 223.17 ±40.93 | 263.96 ±48.21 | 314.03 ±60.9 3 | 373.87 ±74.27 | 419.26 ±80.72 | 518.48 ±105.7 8 | 550.33 ±116. 03 | 604.32 ±130.1 8 | 725.1 8±136 .03 | 767.49 ±116.8 7 | 846.86 ±128.8 5 |
| Group | 146.7 | 194.10 | 138.02 | 124.61 | 105.24 | 107.93 | 110.55 | 123.45 | 101.47 | 114.6 | 134.32 | 146.99 |
| 3 | 6±14. 97 | ±32.47 | ±31.54 | ±25.1 7 | ±22.59 | ±29.10 | ±38.68 | ±43.2 3 | ±32.83 | 9±35. 62 | ±40.59 | ±37.96 |
| Group 4 | 146.0 5±11. 97 | 193.80 ±27.43 | 123.87 ±20.28 | 112.22 ±13.3 6 | 85.08± 10.27 | 59.95± 14.40 | 49.03± 12.67 | 48.35 ±10.5 6 | 65.87± 16.01 | 75.54 ±23.2 0 | 106.10 ±25.73 | 124.68 ±31.35 |
| Group 5 | 147.1 1±13. 81 | 182.60 ±28.23 | 178.72 ±36.66 | 181.66 ±34.3 8 | 181.56 ±36.01 | 156.49 ±37.68 | 152.57 ±41.52 | 152.13 ±42.8 9 | 176.07 ±60.21 | 198.4 2±65. 71 | 221.99 ±73.13 | 263.45 ±86.54 |
| Group 6 | 146.8 1±14. 13 | 213.60 ±33.98 | 233.94 ±55.49 | 277.57 ±68.2 0 | 295.46 ±60.68 | 328.33 ±81.76 | 373.13 ±70.65 | 454.44 ±82.6 7 | 705.45 ±124.0 8 | 886.4 2±166 .06 | 1344.2 6±301. 53 | 1521.3 3±349. 46 |
| Group 7 | 146.8 3±14. 11 | 181.77 ±47.47 | 183.91 ±47.60 | 186.76 ±38.1 6 | 202.47 ±42.51 | 206.06 ±66.25 | 207.63 ±61.32 | 215.23 ±70.4 8 | 267.67 ±99.47 | 295.3 1±123 .02 | 360.39 ±132.0 8 | 378.56 ±157.7 3 |

**Table 14 Efficacy Analysis of Groups in Human Pancreatic Cancer Capan-1 Subcutaneous Model**

| Group | Day 43 after Cell Inoculation | | | | | |
|---|---|---|---|---|---|---|
| | Tumor Volume ($\bar{x}$±S) | Relative Tumor Volume ($\bar{x}$ ±S) | TGI (%) | T/C (%) | P Value (in comparison with control group) | CR Rate |
| Group 1 | 1301.38±76.61 | 9.15±0.70 | - | - | - | |
| Group 2 | 846.86±128.85 | 5.72±0.54 | 37.43 | 62.57 | >0.05 | 0/6 |
| Group 3 | 146.99±37.96 | 0.99±0.22 | 89.17 | 10.83 | <0.001 | 0/6 |
| Group 4 | 124.68±31.35 | 0.83±0.17 | 90.89 | 9.11 | <0.001 | 0/6 |
| Group 5 | 263.45±86.54 | 1.81±0.64 | 80.18 | 19.82 | <0.001 | 2/6 |
| Group 6 | 1521.33±349.46 | 11.12±3.31 | -21.59 | 121.59 | >0.05 | 0/6 |
| Group 7 | 378.56±157.73 | 2.34±0.81 | 74.36 | 25.64 | <0.001 | 0/6 |
| Notes: 1. Data is presented in the form of mean ± standard deviation (STD); 2. T/C % = $T_{RTV}$ / $C_{RTV}$ × 100%; 3. "CR" stands for Complete Response of a tumor (i.e., the size of the tumor diminishes to zero). | | | | | | |

**Table 15 Body Weight Changes of Groups in Human Pancreatic Cancer Capan-1 Subcutaneous Model**

| Test Group | # of Animals | Average Body Weight g ($\bar{x}$±S) | | Body Weight Change Rate (%) | End of Experiment |
|---|---|---|---|---|---|
| | | $6^{th}$ Day after Cell Inoculation (Day 6) | $43^{th}$ Day after Cell Inoculation (Day 43) | $43^{th}$ Day after Cell Inoculation (Day 43) | |
| Group 1 | 6 | 21.2±0.4 | 24.8±0.3 | 17.25%±1.64% | $43^{th}$ day after cell inoculation (Dav 43) |
| Group 2 | 6 | 21.9±0.4 | 24.2±0.4 | 10.95%±2.07% | $43^{th}$ day after cell inoculation (Day 43) |
| Group 3 | 6 | 22.1±0.4 | 24.5±0.3 | 10.74%±1.25% | $43^{th}$ day after cell inoculation (Day 43) |
| Group 4 | 6 | 21.8±0.5 | 24.2±0.7 | 11.04%±0.91 | $43^{th}$ day after cell inoculation (Day 43) |
| Group 5 | 6 | 21.9±0.4 | 25.1±0.4 | 14.81%±0.62 | $43^{th}$ day after cell inoculation (Day 43) |
| Group 6 | 6 | 22.0±0.5 | 26.4±0.7 | 20.46%±3.51 | $43^{th}$ day after cell inoculation (Day 43) |
| Group 7 | 6 | 22.3±0.4 | 25.4±0.5 | 14.59%±1.74 | $43^{th}$ day after cell inoculation (Day 43) |

Findings from the above data are as follows:

The mice in the vehicle control group had an average tumor volume of 1301.38 $mm^3$ on the $43^{th}$ day after tumor cell inoculation (Day 43). On the 43th day after tumor cell inoculation (Day 43), the 100 mg/kg test drug olaparib treatment group (Group 2) had an average tumor volume of 846.86 $mm^3$, and a relative tumor growth inhibition (TGI) rate (%) of 37.43%, without a statistically significant difference (p>0.05) from the control group.

On the 43th day after tumor cell inoculation (Day 43), the 50 mg/kg test drug TH-302 treatment group (Group 3, QD) had an average tumor volume of 146.99 $mm^3$, with a statistically significant difference (p<0.001) from the control group, and a relative tumor growth inhibition (TGI) rate (%) of 89.17%. On the 43th day after tumor cell inoculation (Day

43), the 100 mg/kg test drug TH-302 treatment group (Group 4) had an average tumor volume of 124.68 $mm^3$, with a statistically significant difference ($p<0.001$) from the control group, and a relative tumor growth inhibition (TGI) rate (%) of 90.89%. On the 43th day after tumor cell inoculation (Day 43), the 50 mg/kg test drug TH-302 treatment group (Group 5, QW) had an average tumor volume of 263.45 $mm^3$, with a statistically significant difference ($p<0.001$) from the control group, a relative tumor growth inhibition (TGI) rate (%) of 80.18%, and a complete tumor inhibition rate of 33.3%. On the 43th day after tumor cell inoculation (Day 43), the 25 mg/kg test drug TH-302 treatment group (Group 6) had an average tumor volume of 1521.33 $mm^3$, without a statistically significant difference ($p>0.05$) from the control group, and a relative tumor growth inhibition (TGI) rate (%) of -21.59%. On the 43th day after tumor cell inoculation (Day 43), the 100 mg/kg olaparib + 25 mg/kg TH-302 combination therapy group (Group 7) had an average tumor volume of 378.56 $mm^3$, with a statistically significant difference ($p<0.001$) from the control group, and a relative tumor growth inhibition (TGI) rate (%) of 74.36%.

The 100 mg/kg (Group 4), 50 mg/kg (Group 5, QW) and 25 mg/kg (Group 6) TH-302 monotherapy treatment groups showed dose-dependent tumor growth inhibition effects. Both the 100 mg/kg (Group 4, QW) and 50 mg/kg (Group 5, QW) TH-302 monotherapy treatment groups exhibited a statistically significant difference (in each case, $p<0.001$) from the 25 mg/kg TH-302 monotherapy treatment group (Group 6, QW).

The 100 mg/kg olaparib + 25 mg/kg TH-302 combination therapy group (Group 7) showed a better anti-tumor effect than the 100 mg/kg olaparib (Group 2) and 25 mg/kg TH-302 (Group 6) monotherapy groups, with a statistically significant difference ($p<0.05$ and $p<0.001$) therefrom, demonstrating that the particular dose combination of olaparib and TH-302 in combination therapy created a synergistic tumor growth inhibition effect.

No significant mouse body weight losses were observed in the 100 mg/kg test drug olaparib (Group 2), 25 mg/kg TH-302 (Group 6), 50 mg/kg TH-302 (Group 3, QD), 50 mg/kg TH-302 (Group 5, QW), 100 mg/kg TH-302 (Group 4), 100 mg/kg olaparib + 25 mg/kg TH-302 combination therapy group (Group 7) and the vehicle control group (Group 1), showing good tolerance.

### 3.3 Evaluation of Efficacy and Safety of TH-302 in Olaparib-Resistant Lung Cancer LU6429 PDX Model

The LU6429 PDX model was an olaparib-resistant model with a pathogenic BRCA2 mutation.

Protocol: Nude female Balb/c mice were subcutaneously inoculated with HuPrime® lung cancer LU6429 tumor tissue, establishing a human lung cancer subcutaneous xenograft tumor model. The mice were divided into 6 groups, 6 in each group: 50 mg/kg of the test drug olaparib as a monotherapy (Group 02); 80 mg/kg TH-302 as a monotherapy (Group 03); 40 mg/kg TH-302 as a monotherapy (Group 04); 20 mg/kg TH-302 as a monotherapy (Group 05); 40 mg/kg TH-302 in combination therapy with 50 mg/kg olaparib (Group 06); and a glucose injection solution as a vehicle control (Group 01). The vehicle control group and the TH-302 treatment groups were all administered through tail vein, once weekly for a total of 3 weeks. Olaparib was intragastrically administered once daily for totally 28 days. Routes of administration, doses and regimens for the HuPrime® lung cancer LU6429 animal model are specifically summarized in Table 16.

**Table 16 Routes of Administration, Doses and Regimens for HuPrime® Lung Cancer LU6429 Animal Model**

| Group | # of Animals | Treatment Group | Dose ( mg/ kg ) | Route of Administration | Dosage Cycle |
|---|---|---|---|---|---|
| 1 | 6 | glucose injection solution | - | *i.v.* | QW×3 |
| 2 | 6 | olaparib | 50 | *p.o.* | QD×28 |
| 3 | 6 | TH-302 | 80 | *i.v.* | QW×3 |
| 4 | 6 | TH-302 | 40 | *i.v.* | QW×3 |
| 5 | 6 | TH-302 | 20 | *i.v.* | QW×3 |
| 6 | 6 | olaparib | 50 | *p.o.* | QD×28 |
| | | TH-302 | 40 | *i.v.* | QW×3 |
| Notes: 1. Volumes of administration were 10 μL/g; 2. QD×28: Once daily for consecutive 28 days; 3. QW×3: Once weekly for consecutive 3 weeks. | | | | | |

Tumor growth of the treatment and control groups on different days in the experiment were recorded and shown in Table 17, and corresponding tumor volume growth curves of the mice in respective groups are shown in Fig. 9. Efficacy

evaluation was carried out based on relative tumor proliferation rates and relative tumor growth inhibition rates, and the results of efficacy analysis are summarized in Table 18. Body weight changes of the treatment and control groups after administration were recorded to investigate safety of the groups in the HuPrime® lung cancer LU6429 subcutaneous model. Body weight change rates of the mice on Day 25 are shown in Table 19, and curves showing body weights of the treatment groups over time are plotted in Fig. 10.

**Table 17 Variation of Tumor Volumes of Groups of Mice in HuPrime® Lung Cancer LU6429 Model over Time of Treatment (mm$^3$)**

| Group | Days after Inoculation | | | | | | | | |
|---|---|---|---|---|---|---|---|---|---|
| | Day 0 | Day 4 | Day 7 | Day 11 | Day 14 | Day 18 | Day 21 | Day 25 | Day 27 |
| Group 1 | 102.44±2 .49 | 259.87±1 3.67 | 382.98±3 4.24 | 572.10±6 6.56 | 1007.43± 105.81 | 1337.22± 162.19 | 1765.80± 210.72 | 2256.82± 271.40 | 2362.40 ±274.50 |
| Group 2 | 102.82±2 .80 | 299.24±5 9.50 | 462.91±8 8.04 | 650.87±1 04.16 | 821.67±1 22.23 | 1169.82± 161.72 | 1427.99± 213.12 | 2019.59± 304.46 | 2390.86 ±344.38 |
| Group 3 | 102.99±5 .82 | 173.84±1 7.46 | 191.93±2 0.29 | 161.20±2 0.42 | 126.48±9 .07 | 82.07±13 .35 | 68.83±15 .37 | 60.67±22 .93 | 61.24±2 5.30 |
| Group 4 | 103.27±3 .02 | 227.95±2 3.82 | 273.49±3 1.36 | 347.62±5 0.22 | 324.71±6 0.05 | 368.33±7 3.32 | 351.06±8 3.11 | 409.93±1 13.60 | 466.06± 149.54 |
| Group 5 | 102.03±2 .16 | 305.66±4 5.66 | 426.08±7 9.10 | 570.59±1 17.49 | 640.36±1 26.52 | 812.62±1 54.43 | 893.25±1 47.28 | 1099.39± 251.90 | 1297.75 ±289.08 |
| Group 6 | 103.88±6 .10 | 215.29±2 9.95 | 252.34±3 8.00 | 297.51±4 3.70 | 312.63±4 8.98 | 326.77±4 6.47 | 297.24±5 1.83 | 315.97±5 8.35 | 344.27± 72.07 |

**Table 18 Efficacy Analysis of Groups in HuPrime® Lung Cancer LU6429 Subcutaneous Model**

| Test Group | Day 25 | | | | | |
|---|---|---|---|---|---|---|
| | Tumor Volume ($\bar{x}$±S) | Relative Tumor Volume ($\bar{x}$±S) | TGI (%) | T/C (%) | P Value (in comparison with control group) | Tumor Clearance Rate on Day 27 |
| Group 1 | 2256.82±271.40 | 21.82±2.15 | - | - | - | 0/6 |
| Group 2 | 2019.59±304.46 | 19.69±3.02 | 9.74 | 90.26 | >0.05 | 0/6 |
| Group 3 | 60.67±22.93 | 0.61±0.22 | 97.19 | 2.81 | <0.001*** | 1/6 |
| Group 4 | 409.93±113.60 | 3.99±1.15 | 81.69 | 18.31 | <0.001 *** | 0/6 |
| Group 5 | 1099.39±251.90 | 10.92±2.67 | 49.97 | 50.03 | <0.05* | 0/6 |
| Group 6 | 315.97±58.35 | 2.99±0.48 | 86.31 | 13.69 | <0.001*** | 0/6 |
| Note: Data is presented in the form of mean ± STD. | | | | | | |

**Table 19 Body Weight Changes of Groups in HuPrime® Lung Cancer LU6429 Subcutaneous Model**

| Test Group | # of Animals | Average Body Weight g ($\bar{x}$±S) | | Body Weight Change Rate (%) | End of Experiment |
|---|---|---|---|---|---|
| | | Day 0 | Day 25 | Day 25 | |
| **Group 1** | 6 | 20.6±0.2 | 25.2±0.3 | 22.43%±2.07% | Day 27 |
| **Group 2** | 6 | 20.8±0.2 | 24.0+0.4 | 15.20%±2.31% | Day 27 |
| **Group 3** | 6 | 21.5±0.3 | 23.2±0.2 | 7.94%±0.76% | Day 27 |
| **Group 4** | 6 | 21.6±0.2 | 23.7+0.4 | 10.01%±1.19% | Day 27 |
| **Group 5** | 6 | 20.8±0.2 | 23.2±0.4 | 11.72%±1.73% | Day 27 |
| **Group 6** | 6 | 21.9±0.3 | 23.2+0.3 | 6.03%±0.78% | Day 27 |

Findings from the above data are as follows:
The olaparib monotherapy group did not show a tumor growth inhibition effect, demonstrating resistance of the lung cancer LU6429 PDX model to olaparib. In this study, the 80 mg/kg, 40 mg/kg and 20 mg/kg test drug TH-302 monotherapy treatment groups and the 40 mg/kg TH-302 + 50 mg/kg olaparib combination therapy group all exhibited significant tumor proliferation inhibition effects on the HuPrime® lung cancer LU6429 subcutaneous model. In the 80 mg/kg TH-302 treatment group (Group 03), one mouse experienced complete tumor clearance, resulting in a clearance rate of 16.7%. There are statistically significant differences (p<0.05) among the 80 mg/kg, 40 mg/kg and 20 mg/kg test drug TH-302 treatment groups, suggesting dose dependence. The therapeutic effect of the combination therapy of 40 mg/kg TH-302 with 50 mg/kg olaparib was much better than that of the 50 mg/kg olaparib monotherapy, but only slightly better than that of the 40 mg/kg TH-302 monotherapy with a small difference therefrom, which was insignificant though.

No significant mouse body weight losses were observed in the test drug treatment groups through the treatment period, showing good tolerance.

### 3.4 Evaluation of Efficacy and Safety of TH-302 in Olaparib-Resistant Bladder Cancer BL3325 PDX Model

The BL3325 PDX model was an olaparib-resistant model with a pathogenic BRCA2 mutation.

Protocol: Nude female Balb/c mice were subcutaneously inoculated with HuPrime® bladder cancer BL3325 tumor tissue, establishing a human bladder cancer subcutaneous xenograft tumor model. The mice were divided into 6

groups, 6 in each group: 50 mg/kg of the test drug olaparib as a monotherapy (Group 02); 80 mg/kg TH-302 as a monotherapy (Group 03); 40 mg/kg TH-302 as a monotherapy (Group 04); 20 mg/kg TH-302 as a monotherapy (Group 05); 40 mg/kg TH-302 in combination therapy with 50 mg/kg olaparib (Group 06); and a glucose injection solution as a vehicle control (Group 01). The vehicle control group and the TH-302 treatment groups were all administered through tail vein, once weekly for a total of 3 weeks. Olaparib was intragastrically administered once daily for totally 30 days. Routes of administration, doses and regimens for the HuPrime® bladder cancer BL3325 animal model are specifically summarized in Table 20.

**Table 20 Routes of Administration, Doses and Regimens for HuPrime® Bladder Cancer BL3325 Animal Model**

| Group | # of Animals | Treatment Group | Dose ( mg/ kg ) | Route of Administration | Dosage Cycle |
|---|---|---|---|---|---|
| 1 | 6 | glucose injection solutions | - | *i.v.* | QW×3 |
| 2 | 6 | olaparib | 50 | *p.o.* | QD×30 |
| 3 | 6 | TH-302 | 80 | *i.v.* | QW×3 |
| 4 | 6 | TH-302 | 40 | *i.v.* | QW×3 |
| 5 | 6 | TH-302 | 20 | *i.v.* | QW×3 |
| 6 | 6 | olaparib | 50 | *p.o.* | QD×30 |
| | | TH-302 | 40 | *i.v.* | QW×3 |
| Notes: 1. Volumes of administration were 10 μL/g; 2. QD×30: Once daily for consecutive 30 days; 3. QW×3: Once weekly for consecutive 3 weeks; 4. *i.v.* stands for administration through tail vein; and *p.o.* stands for intragastric administration. | | | | | |

Tumor growth of the treatment and control groups on different days in the experiment were recorded and shown in Table 21, and corresponding tumor volume growth curves of the mice in respective groups are shown in Fig. 11. Efficacy evaluation was carried out based on relative tumor proliferation rates and relative tumor growth inhibition rates, and the results of efficacy analysis are summarized in Table 22. Body weight changes of the treatment and control groups after administration were recorded to investigate safety of the groups in the HuPrime® bladder cancer BL3325 subcutaneous model. Body weight change rates of the mice on Day 35 are shown in Table 23, and curves showing body weights of the treatment groups over time are plotted in Fig. 12.

**Table 21 Variation of Tumor Volumes of Groups of Mice in HuPrime® Bladder Cancer BL3325 Model over Time of Treatment (mm³)**

| Group | Days after Inoculation | | | | | | | | | | |
|---|---|---|---|---|---|---|---|---|---|---|---|
| | Day 0 | Day 4 | Day 7 | Day 11 | Day 14 | Day 18 | Day 21 | Day 25 | Day 28 | Day 32 | Day 35 |
| Group 1 | 128.05 | 247.09 | 366.61 | 590.94 | 769.51 | 1047.80 | 1309.89 | 1716.34 | 1961.01 | 2094.5 7 | 2506.7 0 |
| Group 2 | 127.83 | 225.23 | 303.42 | 410.99 | 525.97 | 661.65 | 854.15 | 1074.23 | 1267.11 | 1493.9 5 | 1717.9 8 |
| Group 3 | 127.79 | 179.28 | 221.96 | 201.30 | 197.43 | 140.80 | 137.34 | 119.36 | 103.28 | 83.34 | 103.73 |
| Group 4 | 127.95 | 190.31 | 251.91 | 298.71 | 294.43 | 276.26 | 268.83 | 259.33 | 260.51 | 342.45 | 464.64 |
| Group 5 | 127.71 | 180.20 | 238.31 | 281.67 | 303.57 | 332.09 | 389.03 | 439.74 | 595.95 | 786.73 | 1028.5 8 |
| Group 6 | 127.93 | 192.20 | 231.88 | 281.35 | 276.14 | 252.53 | 242.19 | 199.69 | 182.06 | 132.96 | 129.97 |

**Table 22 Efficacy Analysis of Groups in HuPrime® Bladder Cancer BL3325 Subcutaneous Model**

| Test Group | 36th Day after Beginning of Administration (Day 35) | | | | | |
|---|---|---|---|---|---|---|
| | Tumor Volume ($\bar{x}\pm S$) | Relative Tumor Volume ($\bar{x}\pm S$) | TGI (%) | T/C (%) | P Value (in comparison with control group) | Tumor Clearance Rate |
| Group 1 | 2506.70±403.02 | 19.30±2.69 | / | / | - | 0/6 |
| Group 2 | 1717.98±129.57 | 13.43±0.40 | 30.42 | 69.58 | >0.05 | 0/6 |
| Group 3 | 103.73±38.44 | 0.76±20.26 | 96.05 | 3.95 | <0.001*** | 1/6 |
| Group 4 | 464.64±168.62 | 3.78±1.60 | 80.42 | 19.58 | <0.001*** | 0/6 |
| Group 5 | 1028.58±111.81 | 8.08±0.81 | 58.16 | 41.84 | <0.05** | 0/6 |
| Group 6 | 129.97±9.59 | 1.03±0.09 | 94.66 | 5.34 | <0.001*** | 0/6 |
| Notes: 1. Data is presented in the form of mean ± STD; 2. T/C % = $T_{RTV}$ / $C_{RTV}$ * 100%. | | | | | | |

**Table 23 Body Weight Changes of Groups in HuPrime® Bladder Cancer BL3325 Subcutaneous Model**

| Test Group | # of animals at Beginning/End of Experiment | Average Body Weight g ($\bar{x}\pm S$) | | Body Weight Change Rate (%) | End of Experiment |
|---|---|---|---|---|---|
| | | Day 0 | Day 35 | Day 35 | |
| **Group 1** glucose injection solution, 10 μL/g, *i.v.*, QW×3 | 6/6 | 21.5±0.4 | 22.2±0.5 | 3.19%±2.78% | Day 35 |
| **Group 2** Olaparib, 50 mg/kg, 10 μL/g, *p.o.*, QD×30 | 6/6 | 21.8±0.5 | 20.9±0.7 | -4.24%±2.93% | Day 35 |
| **Group 3** TH-302, 80 mg/kg, 10 μL/g, *i.v.*, QW×3 | 6/6 | 21.7±0.4 | 23.8±0.6 | 9.46%±0.99% | Day 35 |
| **Group 4** TH-302, 40 mg/kg, 10 μL/g, *i.v.*, QW×3 | 6/6 | 21.5±0.3 | 23.2±0.2 | 7.88%±1.16% | Day 35 |
| **Group 5** TH-302, 20 mg/kg, 10 μL/g, *i.v.*, QW×3 | 6/6 | 21.6±0.3 | 23.0±0.5 | 6.30%±1.07% | Day 35 |
| **Group 6** TH-302, 40 mg/kg, 10 μL/g, *i.v.*, QW×3 + olaparib, 50 mg/kg, 10 μL/g, *p.o.*, QD×30 | 6/6 | 21.6±0.2 | 22.6±0.4 | 4.37%±0.98% | Day 35 |
| Note: Data is presented in the form of mean ± STD. | | | | | |

Findings from the above data are as follows:
The 80 mg/kg, 40 mg/kg and 20 mg/kg test drug TH-302 monotherapy treatment groups and the 40 mg/kg TH-302 + 50 mg/kg olaparib combination therapy group all exhibited significant tumor proliferation inhibition effects on the HuPrime® bladder cancer BL3325 subcutaneous model, while the 50 mg/kg olaparib monotherapy group did not show a significant tumor growth inhibition effect. There was a statistical difference between the tumor growth inhibition effects of the 80 mg/kg and 20 mg/kg TH-302 treatment groups, demonstrating dose dependence of TH-302 inhibition against BL3325 tumor growth. In the 80 mg/kg TH-302 treatment group (Group 03), one mouse experienced complete tumor clearance, resulting in a clearance rate of 16.7%. The therapeutic effect of the combination therapy of 40 mg/kg TH-302 with 50 mg/kg olaparib was much better than that of the 50 mg/kg olaparib monotherapy, and was better than that of the 40 mg/kg TH-302 monotherapy with a significant difference therefrom.

No significant mouse body weight losses were observed in the test drug treatment groups through the treatment period, showing good tolerance.

Although olaparib was used as a PARP inhibitor in the Examples disclosed herein, rucaparib, niraparib, talazoparib, fluzoparib, pamiparib and other PARP inhibitors all have a similar mechanism of action to olaparib, i.e., blocking an enzyme involved in repair of damaged DNA from playing its role. Therefore, it is contemplated that rucaparib, niraparib, talazoparib, fluzoparib, pamiparib and other PARPis have similar tumor inhibition efficacy to that of olaparib used in the above experiments.

TH-302 is a hypoxia-activated DNA alkylator, and compounds of the general formula set forth in Claim 1:

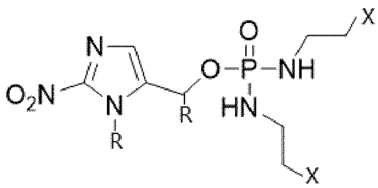

(I)

have been proven in related patent applications to have a similar mechanism of action to TH-302. Therefore, it is totally predictable that these compounds can offer similar effects to TH-302.

## Claims

1. A treatment method, using a drug containing a hypoxia activated compound of formula (I) as a monotherapy or in combination therapy to treat a PARP inhibitor-resistant cancer or tumor patient:

   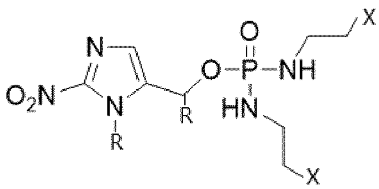

   (I),

   where each R is independently selected from H, $-CH_3$ and $-CH_2CH_3$, and each X is independently selected from Cl, Br, MsO, TsO and other leaving functional groups.

2. The treatment method according to claim 1, using a drug containing a hypoxia activated compound of formula (I) in combination therapy with a PARP inhibitor to treat a PARP inhibitor-resistant cancer or tumor patient.

3. The treatment method according to claim 1 or 2, wherein,

   a DNA repair enzyme in the patient is impaired; or
   any one or two of the genes corresponding to BRCA1/BRCA2 have been determined to be mutated based on the detection of the tumor or cancer tissue of the patient; or
   any one or two of the genes corresponding to BRCA1/BRCA2 have been determined to be mutated based on the detection the patient.

4. The treatment method according to claim 3, wherein the BRCA1/BRCA2 mutation(s) include(s) germline (gBRCAm) and somatic (sBRCAm) BRCA1/BRCA2 mutation(s).

5. The treatment method according to claim 1 or 2, wherein,

the PARP inhibitor is selected from olaparib, rucaparib, niraparib, talazoparib, fluzoparib and pamiparib; and the cancer or tumor is selected from ovarian cancer, breast cancer, pancreatic cancer, fallopian tube cancer, primary peritoneal cancer, gastric cancer, prostate cancer, liver cancer, colon cancer, rectal cancer, lung cancer and bladder cancer, and the lung cancer is preferred to be non-small-cell lung cancer or small-cell lung cancer.

6. The treatment method according to claim 1 or 2, wherein the hypoxia activated compound of formula (I) is selected from a compound of the following structure:

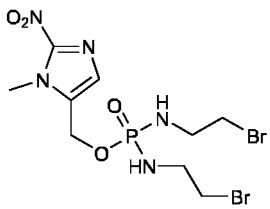

.

7. A treatment method, using a drug containing a hypoxia activated compound of the following formula as a monotherapy to treat an olaparib-resistant patient with ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer or bladder cancer:

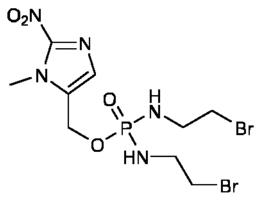

,

wherein any one or two of the genes corresponding to BRCA1/BRCA2 have been determined to be mutated based on the detection of the tumor or cancer tissue of the patient; or any one or two of the genes corresponding to BRCA1/BRCA2 have been determined to be mutated based on the detection of the patient.

8. A treatment method, which uses a drug containing a hypoxia activated compound of the following formula in combination therapy with olaparib to treat an olaparib-resistant patient with ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer or bladder cancer:

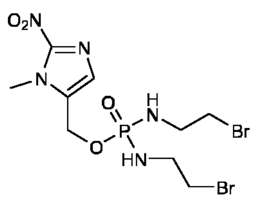

,

wherein any one or two of the genes corresponding to BRCA1/BRCA2 have been determined to be mutated based on the detection of the tumor or cancer tissue of the patient; or any one or two of the genes corresponding to BRCA1/BRCA2 have been determined to be mutated based on the detection of the patient.

9. A treatment method, comprising the steps of:

detecting a BRCA1/BRCA2 genetic mutation condition of a PARP inhibitor-resistant cancer or tumor patient;

if there is a BRCA1/BRCA2 genetic mutation in the patient, using a drug containing a hypoxia activated compound of formula (I) as a monotherapy or in combination therapy with a PARP inhibitor for treatment:

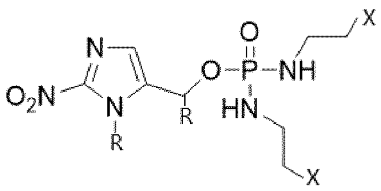 (I),

where each R is independently selected from H, $-CH_3$ and $-CH_2CH_3$, and each X is independently selected from Cl, Br, MsO, TsO and other leaving functional groups.

**10.** The treatment method according to any of claim 3, or 4, or 7, or 8, or 9, wherein the genetic mutation(s) has(ve) a medium tumor mutational burden (TMB) level.

**11.** Use of a hypoxia activated compound of formula (I) for preparing a drug for treating cancer in a patient as a monotherapy or in combination therapy with a PARP inhibitor:

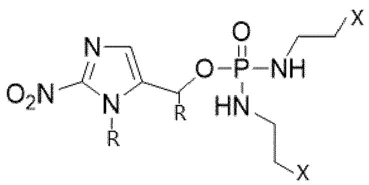 (I),

wherein the patient is a PARP inhibitor-resistant patient; and
each R is independently selected from H, $-CH_3$ and $-CH_2CH_3$, and each X is independently selected from Cl, Br, MsO, TsO and other leaving functional groups.

**12.** The use according to claim 11, wherein

a DNA repair enzyme in the patient is impaired; or
any one or two of the genes corresponding to BRCA1/BRCA2 have been determined to be mutated based on the detection of the tumor or cancer tissue of the patient; or
any one or two of the genes corresponding to BRCA1/BRCA2 have been determined to be mutated based on the detection of the patient.

**13.** The use according to claim 12, wherein

the BRCA1/BRCA2 mutation(s) include(s) germline (gBRCAm) and somatic (sBRCAm) BRCA1/BRCA2 mutation(s); and
the genetic mutation(s) has(ve) a medium tumor mutational burden (TMB) level.

**14.** The use according to claim 11, wherein the hypoxia activated compound of formula (I) is selected from a compound of the following structure:

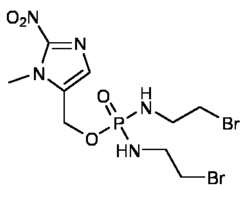 ;

or

the PARP inhibitor is selected from olaparib, rucaparib, niraparib, talazoparib, fluzoparib and pamiparib; or
the cancer or tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer and bladder cancer.

**15.** A drug for treating a PARP inhibitor-resistant cancer or tumor patient, the drug containing a hypoxia activated compound of formula (I):

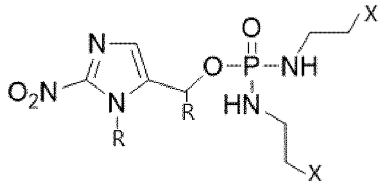
(I),

where each R is independently selected from H, $-CH_3$ and $-CH_2CH_3$, and each X is independently selected from Cl, Br, MsO, TsO and other leaving functional groups.

**16.** The drug according to claim 15, wherein

a DNA repair enzyme in the patient is impaired; or
any one or two of the genes corresponding to BRCA1/BRCA2 have been determined to be mutated based on the detection of the tumor or cancer tissue of the patient; or
any one or two of the genes corresponding to BRCA1/BRCA2 have been determined to be mutated based on the detection of the patient, and preferably, the BRCA1BRCA2 mutation(s) include(s) germline (gBRCAm) and somatic (sBRCAm) BRCA1/BRCA2 mutation(s).

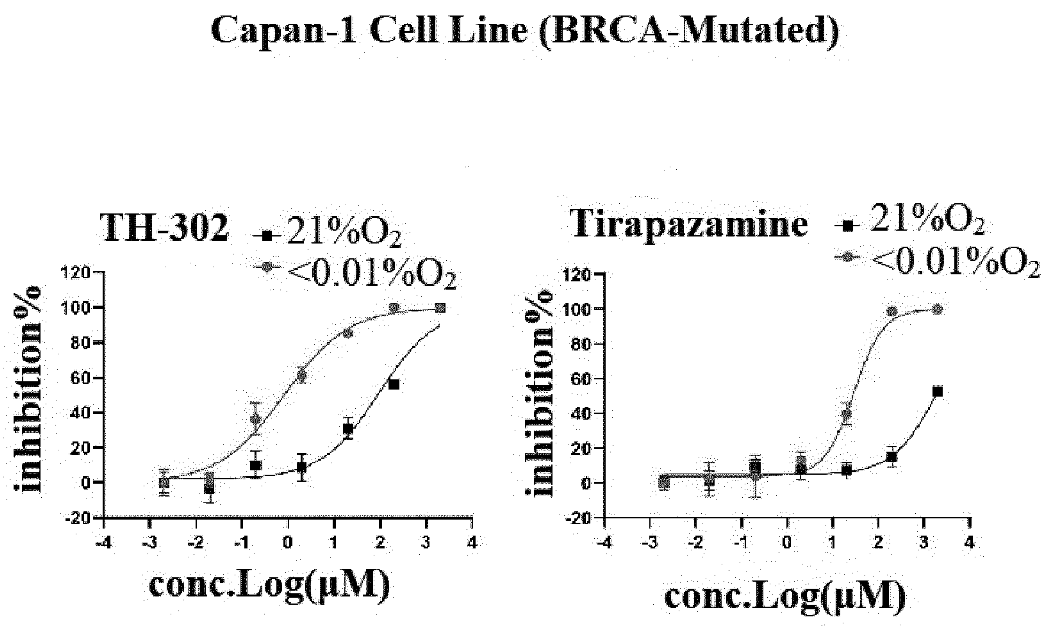
Capan-1 Cell Line (BRCA-Mutated)
TH-302
21%O2
<0.01%O2
Tirapazamine
21%O2
<0.01%O2
inhibition%
conc.Log(μM)
inhibition%
conc.Log(μM)

Fig. 1

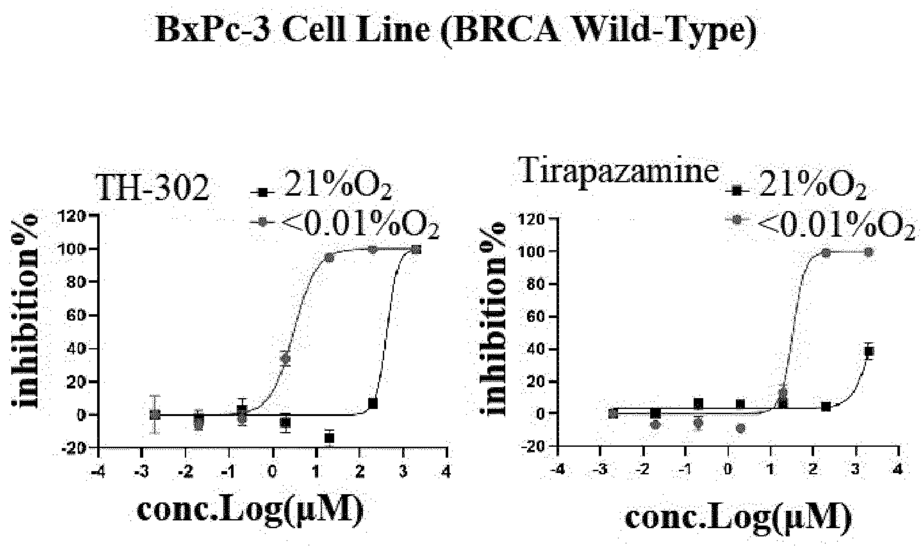
BxPc-3 Cell Line (BRCA Wild-Type)
TH-302
21%O2
<0.01%O2
Tirapazamine
21%O2
<0.01%O2
inhibition%
conc.Log(μM)
inhibition%
conc.Log(μM)

Fig. 2

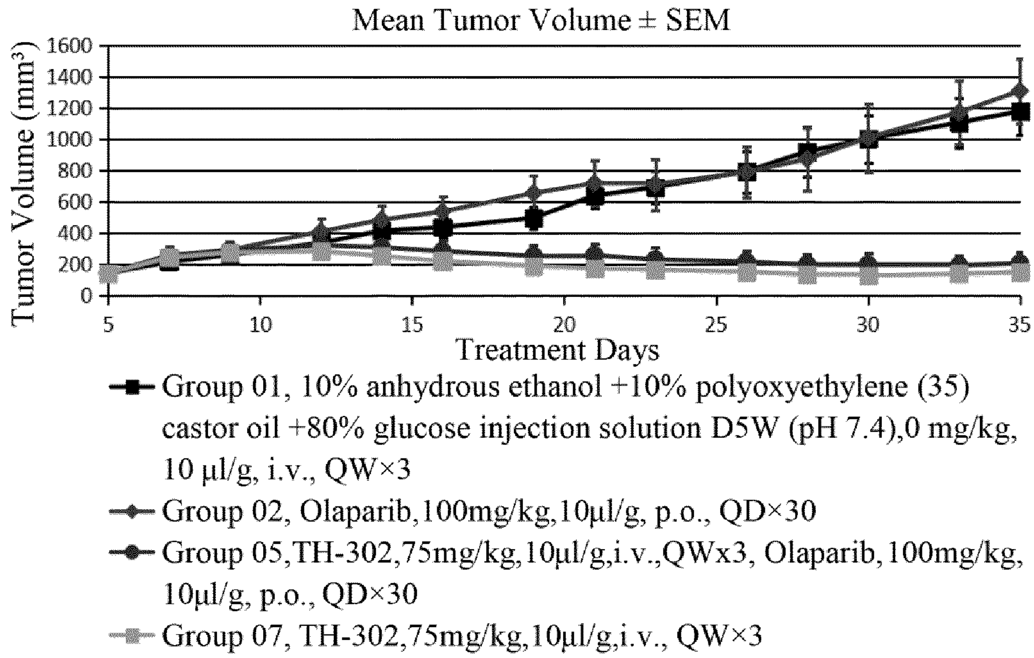
Mean Tumor Volume ± SEM
Tumor Volume (mm³)
1600
1400
1200
1000
800
600
400
200
0
5
10
15
20
25
30
35
Treatment Days
Group 01, 10% anhydrous ethanol +10% polyoxyethylene (35) castor oil +80% glucose injection solution D5W (pH 7.4),0 mg/kg, 10 µl/g, i.v., QW×3
Group 02, Olaparib,100mg/kg,10µl/g, p.o., QD×30
Group 05,TH-302,75mg/kg,10µl/g,i.v.,QWx3, Olaparib,100mg/kg, 10µl/g, p.o., QD×30
Group 07, TH-302,75mg/kg,10µl/g,i.v., QW×3

Fig. 3

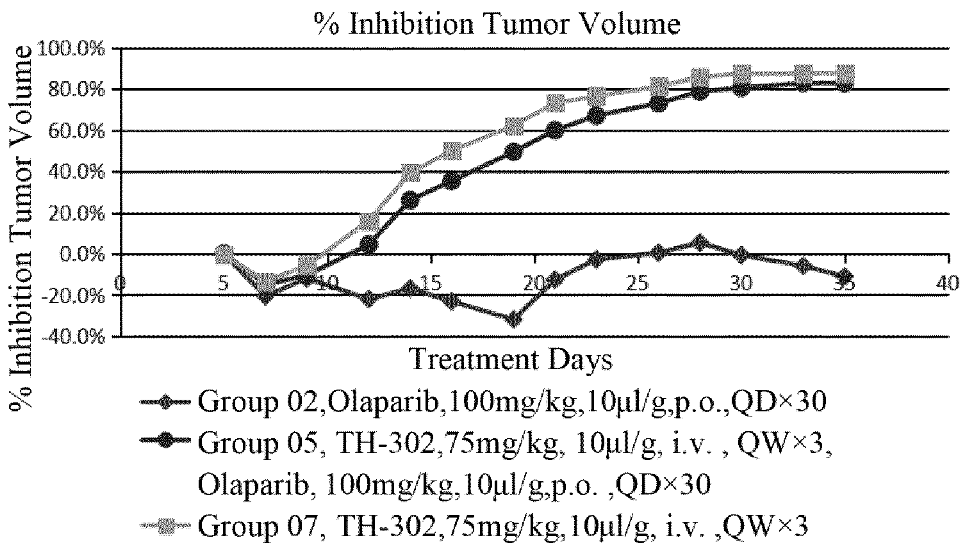
% Inhibition Tumor Volume
% Inhibition Tumor Volume
100.0%
80.0%
60.0%
40.0%
20.0%
0.0%
-20.0%
-40.0%
0
5
10
15
20
25
30
35
40
Treatment Days
Group 02,Olaparib,100mg/kg,10µl/g,p.o.,QD×30
Group 05, TH-302,75mg/kg, 10µl/g, i.v. , QW×3, Olaparib, 100mg/kg,10µl/g,p.o. ,QD×30
Group 07, TH-302,75mg/kg,10µl/g, i.v. ,QW×3

Fig. 4

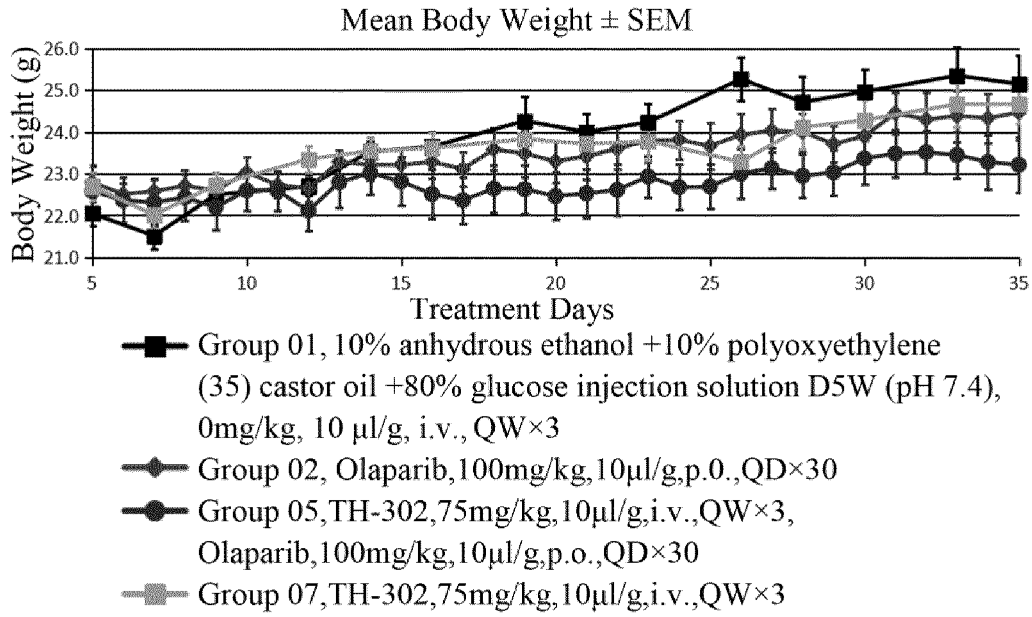
Mean Body Weight ± SEM
Body Weight (g)
26.0
25.0
24.0
23.0
22.0
21.0
5
10
15
20
25
30
35
Treatment Days
Group 01, 10% anhydrous ethanol +10% polyoxyethylene (35) castor oil +80% glucose injection solution D5W (pH 7.4), 0mg/kg, 10 μl/g, i.v., QW×3
Group 02, Olaparib,100mg/kg,10μl/g,p.0.,QD×30
Group 05,TH-302,75mg/kg,10μl/g,i.v.,QW×3, Olaparib,100mg/kg,10μl/g,p.o.,QD×30
Group 07,TH-302,75mg/kg,10μl/g,i.v.,QW×3

Fig. 5

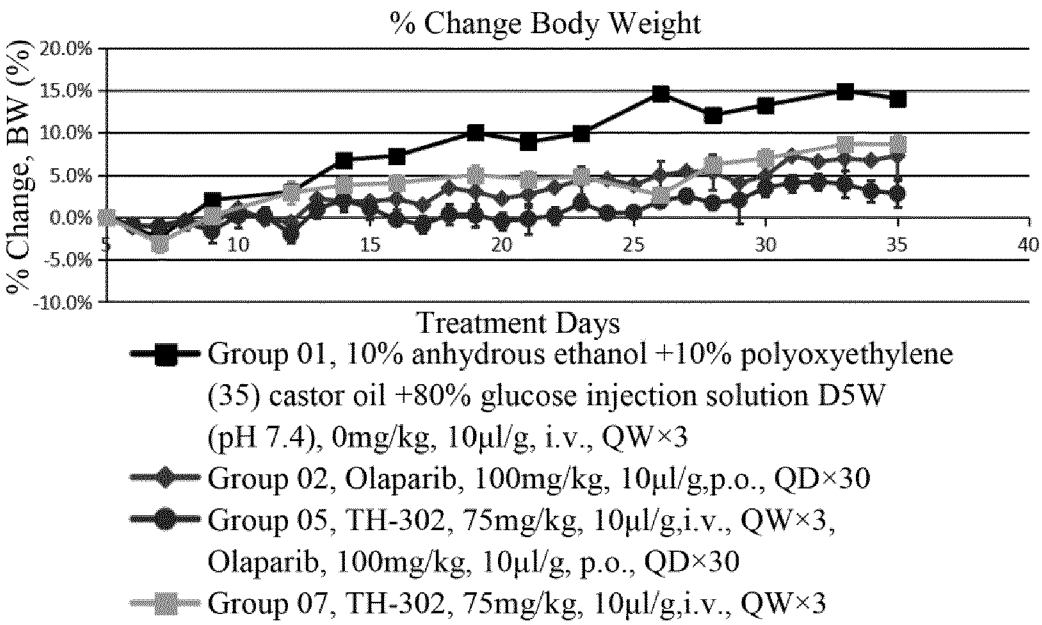
% Change Body Weight
% Change, BW (%)
20.0%
15.0%
10.0%
5.0%
0.0%
-5.0%
-10.0%
5
10
15
20
25
30
35
40
Treatment Days
Group 01, 10% anhydrous ethanol +10% polyoxyethylene (35) castor oil +80% glucose injection solution D5W (pH 7.4), 0mg/kg, 10μl/g, i.v., QW×3
Group 02, Olaparib, 100mg/kg, 10μl/g,p.o., QD×30
Group 05, TH-302, 75mg/kg, 10μl/g,i.v., QW×3, Olaparib, 100mg/kg, 10μl/g, p.o., QD×30
Group 07, TH-302, 75mg/kg, 10μl/g,i.v., QW×3

Fig. 6

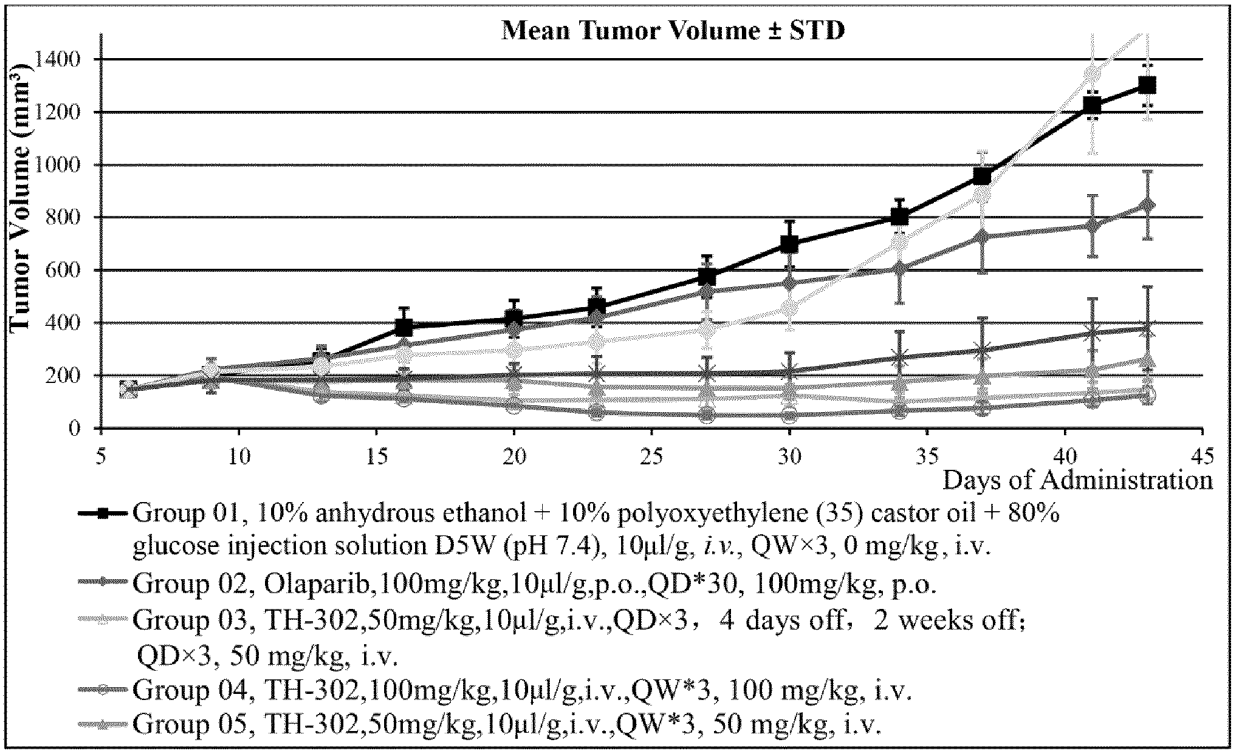
Mean Tumor Volume ± STD
Tumor Volume (mm³)
1400
1200
1000
800
600
400
200
0
5 10 15 20 25 30 35 40 45
Days of Administration
Group 01, 10% anhydrous ethanol + 10% polyoxyethylene (35) castor oil + 80% glucose injection solution D5W (pH 7.4), 10μl/g, i.v., QW×3, 0 mg/kg, i.v.
Group 02, Olaparib,100mg/kg,10μl/g,p.o.,QD*30, 100mg/kg, p.o.
Group 03, TH-302,50mg/kg,10μl/g,i.v.,QD×3, 4 days off, 2 weeks off; QD×3, 50 mg/kg, i.v.
Group 04, TH-302,100mg/kg,10μl/g,i.v.,QW*3, 100 mg/kg, i.v.
Group 05, TH-302,50mg/kg,10μl/g,i.v.,QW*3, 50 mg/kg, i.v.

Fig. 7

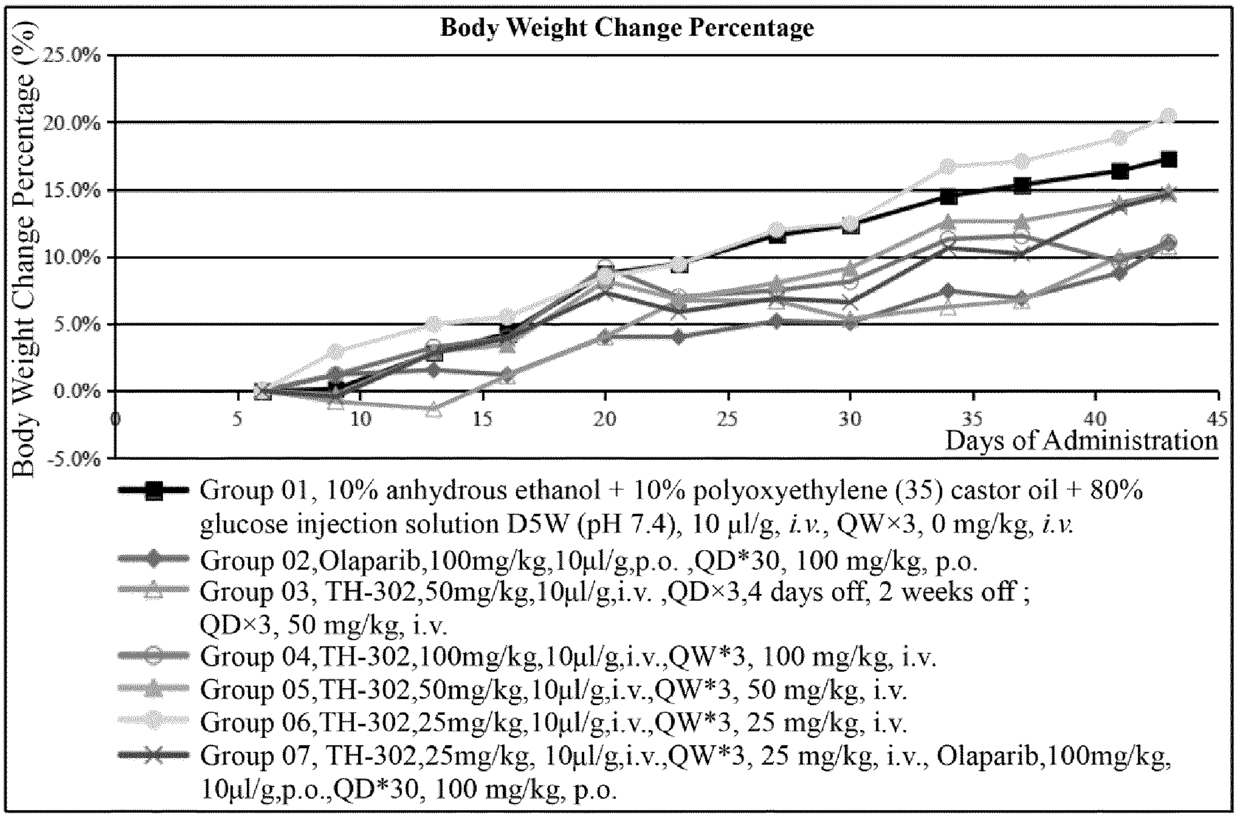
Body Weight Change Percentage
Body Weight Change Percentage (%)
25.0%
20.0%
15.0%
10.0%
5.0%
0.0%
-5.0%
0 5 10 15 20 25 30 35 40 45
Days of Administration
Group 01, 10% anhydrous ethanol + 10% polyoxyethylene (35) castor oil + 80% glucose injection solution D5W (pH 7.4), 10 μl/g, i.v., QW×3, 0 mg/kg, i.v.
Group 02,Olaparib,100mg/kg,10μl/g,p.o. ,QD*30, 100 mg/kg, p.o.
Group 03, TH-302,50mg/kg,10μl/g,i.v. ,QD×3,4 days off, 2 weeks off ; QD×3, 50 mg/kg, i.v.
Group 04,TH-302,100mg/kg,10μl/g,i.v.,QW*3, 100 mg/kg, i.v.
Group 05,TH-302,50mg/kg,10μl/g,i.v.,QW*3, 50 mg/kg, i.v.
Group 06,TH-302,25mg/kg,10μl/g,i.v.,QW*3, 25 mg/kg, i.v.
Group 07, TH-302,25mg/kg, 10μl/g,i.v.,QW*3, 25 mg/kg, i.v., Olaparib,100mg/kg, 10μl/g,p.o.,QD*30, 100 mg/kg, p.o.

Fig. 8

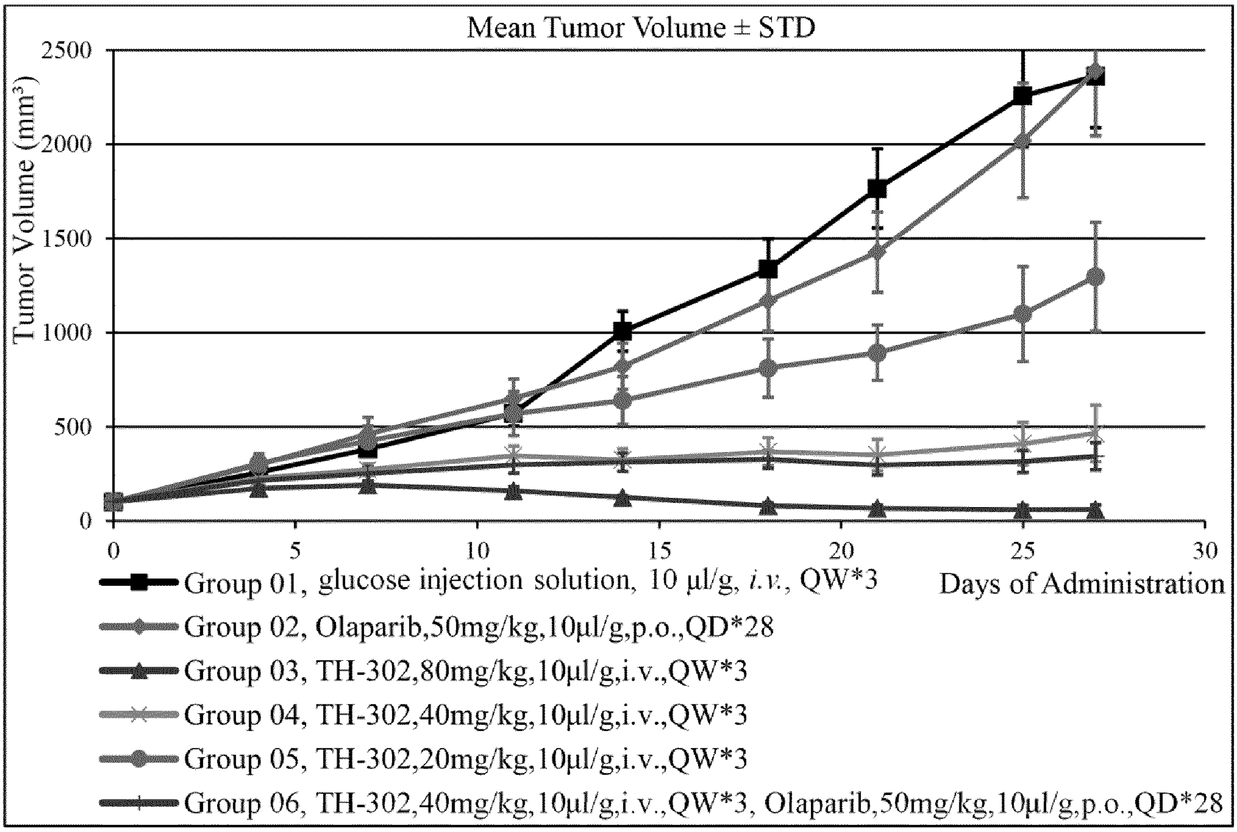
Mean Tumor Volume ± STD
Tumor Volume (mm³)
2500
2000
1500
1000
500
0
0
5
10
15
20
25
30
Days of Administration
Group 01, glucose injection solution, 10 μl/g, i.v., QW*3
Group 02, Olaparib,50mg/kg,10μl/g,p.o.,QD*28
Group 03, TH-302,80mg/kg,10μl/g,i.v.,QW*3
Group 04, TH-302,40mg/kg,10μl/g,i.v.,QW*3
Group 05, TH-302,20mg/kg,10μl/g,i.v.,QW*3
Group 06, TH-302,40mg/kg,10μl/g,i.v.,QW*3, Olaparib,50mg/kg,10μl/g,p.o.,QD*28

Fig. 9

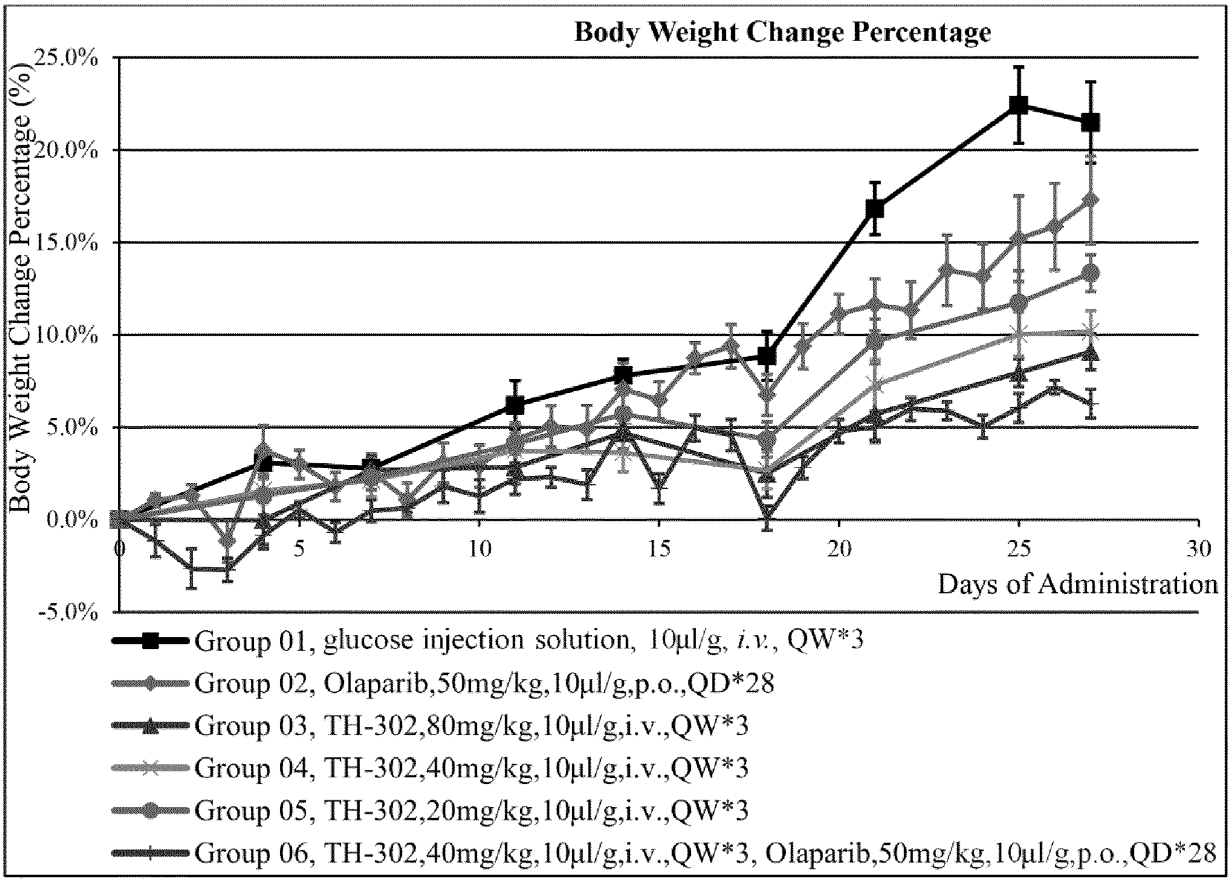
Body Weight Change Percentage
Body Weight Change Percentage (%)
25.0%
20.0%
15.0%
10.0%
5.0%
0.0%
-5.0%
0
5
10
15
20
25
30
Days of Administration
Group 01, glucose injection solution, 10μl/g, i.v., QW*3
Group 02, Olaparib,50mg/kg,10μl/g,p.o.,QD*28
Group 03, TH-302,80mg/kg,10μl/g,i.v.,QW*3
Group 04, TH-302,40mg/kg,10μl/g,i.v.,QW*3
Group 05, TH-302,20mg/kg,10μl/g,i.v.,QW*3
Group 06, TH-302,40mg/kg,10μl/g,i.v.,QW*3, Olaparib,50mg/kg,10μl/g,p.o.,QD*28

Fig. 10

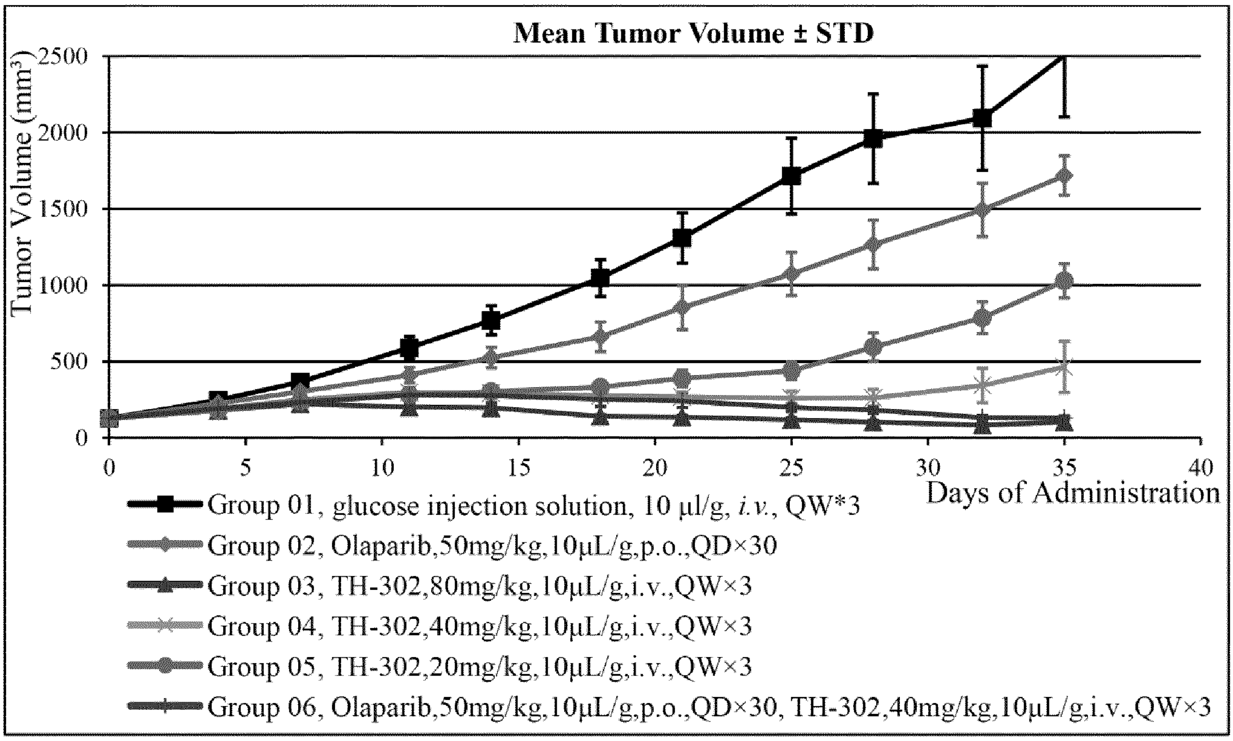
Mean Tumor Volume ± STD
Tumor Volume (mm³)
2500
2000
1500
1000
500
0
0
5
10
15
20
25
30
35
40
Days of Administration
Group 01, glucose injection solution, 10 μl/g, i.v., QW*3
Group 02, Olaparib,50mg/kg,10μL/g,p.o.,QD×30
Group 03, TH-302,80mg/kg,10μL/g,i.v.,QW×3
Group 04, TH-302,40mg/kg,10μL/g,i.v.,QW×3
Group 05, TH-302,20mg/kg,10μL/g,i.v.,QW×3
Group 06, Olaparib,50mg/kg,10μL/g,p.o.,QD×30, TH-302,40mg/kg,10μL/g,i.v.,QW×3

Fig. 11

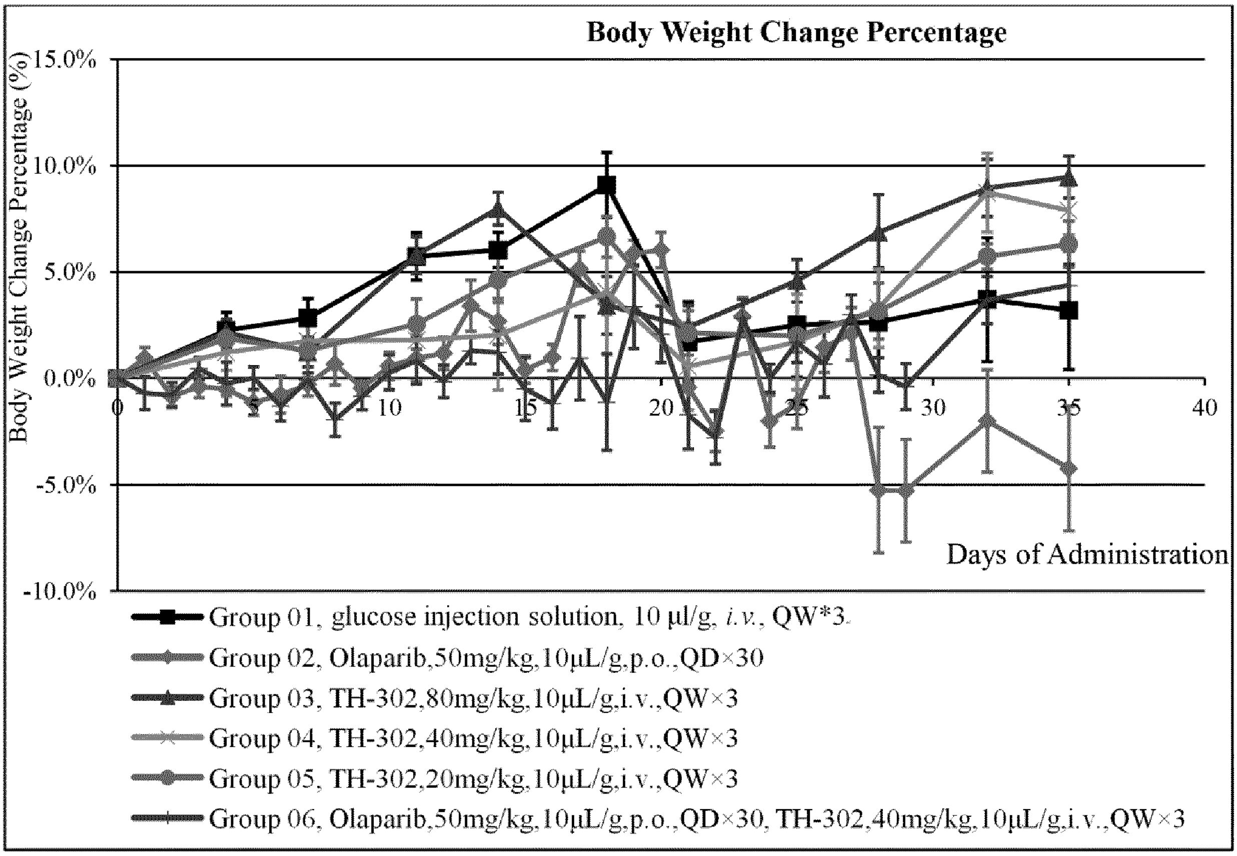
Body Weight Change Percentage
Body Weight Change Percentage (%)
15.0%
10.0%
5.0%
0.0%
-5.0%
-10.0%
0
5
10
15
20
25
30
35
40
Days of Administration
Group 01, glucose injection solution, 10 μl/g, i.v., QW*3
Group 02, Olaparib,50mg/kg,10μL/g,p.o.,QD×30
Group 03, TH-302,80mg/kg,10μL/g,i.v.,QW×3
Group 04, TH-302,40mg/kg,10μL/g,i.v.,QW×3
Group 05, TH-302,20mg/kg,10μL/g,i.v.,QW×3
Group 06, Olaparib,50mg/kg,10μL/g,p.o.,QD×30, TH-302,40mg/kg,10μL/g,i.v.,QW×3

Fig. 12

## INTERNATIONAL SEARCH REPORT

International application No.
**PCT/CN2022/115284**

**A. CLASSIFICATION OF SUBJECT MATTER**

A61K 31/675(2006.01)i；A61P 35/00(2006.01)i

According to International Patent Classification (IPC) or to both national classification and IPC

**B. FIELDS SEARCHED**

Minimum documentation searched (classification system followed by classification symbols)

A61K，A61P

Documentation searched other than minimum documentation to the extent that such documents are included in the fields searched

Electronic data base consulted during the international search (name of data base and, where practicable, search terms used)

CNABS; DWPI; VEN; CNTXT; WOTXT; EPTXT; USTXT; CNKI; 万方数据知识服务平台; WANFANG DATA KNOWLEDGE SERVICE PLATFORM, STN; Web of Science; Elsevier Science Direct; PubMed; 百度学术; BAIDU SCHOLAR: 式I化合物结构式检索, structural formula search for a compound of formula I, TH302, 埃夫索胺, Evofosfamide, CAS918633-87-1, PARP抑制剂, BRCA, 奥拉帕利, 芦卡帕利, 尼拉帕利, 他拉唑帕利, 氟唑帕利, 帕米帕利, Olaparib, Rucaparib, Niraparib, Talazoparib, Fluzoparib, Pamiparib, gBRCAm, sBRCAm

**C. DOCUMENTS CONSIDERED TO BE RELEVANT**

| Category* | Citation of document, with indication, where appropriate, of the relevant passages | Relevant to claim No. |
|---|---|---|
| X | WO 2020118251 A2 (THE BOARD OF THUSTEES OF THE LELAND STANFORD JUNIOR UNIVERSITY) 11 June 2020 (2020-06-11)<br>claims 3-6, and description, paragraphs 0005-0010 | 1-16 |
| X | WO 2012135757 A2 (THRESHOLD PHARMACEUTICALS, INC.) 04 October 2012 (2012-10-04)<br>claims 6-7, and description, paragraphs 0047-0053, and embodiment 1 | 1-16 |
| X | VENA, F. et al. "MEK inhibition leads to BRCA2 downregulation and sensitization to DNA damaging agents in pancreas and ovarian cancer models"<br>*Oncotarget,* Vol. 9, No. 14, 22 January 2018 (2018-01-22),<br>pp. 11592-11603 | 1-16 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 November 2022** | **25 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/115284**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-10**
because they relate to subject matter not required to be searched by this Authority, namely:

[1] Claims 1-10 relate to a method for treating diseases, belonging to the subject matter for which no search is required (PCT Rule 39.1(iv)). However, the present report still relates to performing a search on a corresponding pharmaceutical application.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/115284**

| Patent document cited in search report | Publication date (day/month/year) | Patent family member(s) | Publication date (day/month/year) |
|---|---|---|---|
| WO 2020118251 A2 | 11 June 2020 | WO 2020118251 A3 | 16 July 2020 |
| | | TW 202039002 A | 01 November 2020 |
| | | WO 2020118251 A9 | 21 January 2021 |
| | | US 2022249522 A1 | 11 August 2022 |
| WO 2012135757 A2 | 04 October 2012 | WO 2012135757 A3 | 29 November 2012 |
| | | AU 2012236142 A1 | 17 October 2013 |
| | | IL 228644 A | 28 November 2013 |
| | | CN 103458896 A | 18 December 2013 |
| | | MX 2013011199 A | 31 December 2013 |
| | | EP 2694062 A2 | 12 February 2014 |
| | | KR 20140038390 A | 28 March 2014 |
| | | JP 2014509658 A | 21 April 2014 |
| | | US 2014171389 A1 | 19 June 2014 |
| | | EP 2694062 A4 | 12 November 2014 |
| | | RU 2013146659 A | 10 May 2015 |
| | | CN 103458896 B | 10 February 2016 |
| | | BR 112013024730 A2 | 20 December 2016 |
| | | CA 2831612 A1 | 04 October 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015013448 A1
- WO 2020007106 A1
- US 2012031677 W
- WO 2012135757 A2
- US 2019065065 W
- WO 2020118251 A2
- WO 2010048330 A1
- WO 2012142520 A2
- WO 2008083101 A1
- WO 2016011195 A2
- WO 2004087075 A1
- WO 2007002931 A1
- WO 2008151253 A2
- WO 2009018163 A1
- WO 2009033165 A2
- WO 2010048330 A2
- WO 2012142520 A1
- WO 2008083101 A2
- WO 2020118251 A1
- WO 2014169035 A1
- WO 2013116385 A1
- WO 2019173799 A2
- WO 2016081547 A1
- WO 2014062856 A1
- WO 2015069489 A1
- WO 2012006032 A2
- WO 2018026606 A2
- WO 2015171647 A1
- WO 2013096687 A1
- WO 2013126539 A2
- WO 2013096684 A2
- WO 2012009288 A2
- WO 2012145684 A2
- WO 2016014390 A2
- WO 2019055786 A2
- WO 2015013448 A2
- WO 2016011328 A2
- WO 2013177633 A2
- WO 2015051921 A2

### Non-patent literature cited in the description


- **ASHWORTH, A. ; LORD, C. J.** Synthetic Lethal Therapies for Cancer: What's Next after PARP Inhibitors?. *Nature Reviews. Clinical Oncology,* 2018, vol. 15 (9), 564-576, https://doi.org/10.1038/s41571-018-0055-6
- **MATEO, J. ; LORD, C. J. ; SERRA, V ; TUTT, A. ; BALMAÑA, J. ; CASTROVIEJO-BERMEJO, M. ; CRUZ, C. ; OAKNIN, A. ; KAYE, S. B. ; DE BONO, J. S.** A Decade of Clinical Development of PARP Inhibitors in Perspective. *Annals of Oncology: official journal of the European Society for Medical Oncology,* 2019, vol. 30 (9), 1437-1447, https://doi.org/10.1093/annonc/mdz192
- **HE LI ; ZHAO-YI LIU ; NAYIYUAN WU ; YONG-CHANG CHEN ; QUAN CHENG ; JING WANG.** *PARP Inhibitor Resistance: the Underlying Mechanisms and Clinical Implications. Mol Cancer,* 20 June 2020, vol. 19 (1), 107, https://doi.org/10.1186/s12943-020-01227-0
- **ROSE, M. ; BURGESS, J. T. ; O'BYRNE, K. ; RICHARD, D. J. ; BOLDERSON, E.** PARP Inhibitors: Clinical Relevance, Mechanisms of Action and Tumor Resistance. *Frontiers in Cell and Developmental Biology,* 2020, vol. 8, 564601, https://doi.org/10.3389/fcell.2020.564601
- *CHEMICAL ABSTRACTS,* 918633-87-1